(19) Europäisches Patentamt
European Patent Office
Office européen des brevets

(11) EP 0 690 052 B1

(12) **EUROPÄISCHE PATENTSCHRIFT**

(45) Veröffentlichungstag und Bekanntmachung des Hinweises auf die Patenterteilung:
**24.11.2004 Patentblatt 2004/48**

(51) Int Cl.$^7$: **C07D 239/26**, C09K 11/06

(21) Anmeldenummer: **95109928.2**

(22) Anmeldetag: **26.06.1995**

(54) **Verwendung von Pyrimidingruppen enthaltenden konjugierten Verbindungen als Elektrolumineszenzmaterialien**

Use of pyrimidine groups containing conjugated compounds as electroluminescence materials

Application de composés conjugués contenant des groupements pyrimidines comme matériaux d'électroluminescence

(84) Benannte Vertragsstaaten:
**AT CH DE ES FR GB IT LI NL SE**

(30) Priorität: **01.07.1994 DE 4423098**

(43) Veröffentlichungstag der Anmeldung:
**03.01.1996 Patentblatt 1996/01**

(73) Patentinhaber: **Covion Organic Semiconductors GmbH**
**65926 Frankfurt (DE)**

(72) Erfinder:
- **Gompper, Rudolf, Prof. Dr.**
  **D-81247 München (DE)**
- **Brandl, Stefan, Dr.**
  **D-85521 Riemerling (DE)**
- **Mair, Hans-Jürgen, Dr.**
  **D-86316 Friedberg (DE)**

(74) Vertreter: **Luderschmidt, Schüler & Partner GbR**
**Patentanwälte**
**Industriepark Höchst,**
**Gebäude F 821**
**65926 Frankfurt (DE)**

(56) Entgegenhaltungen:
**EP-A- 0 160 790**
**EP-A- 0 186 045**
**EP-A- 0 373 582**
**EP-A- 0 387 715**
**WO-A-93/06192**
**DE-A- 4 409 431**
**US-A- 4 539 507**

- **CHEMICAL ABSTRACTS, vol. 114, no. 7, 18.Februar 1991 Columbus, Ohio, US; abstract no. 62052z, Seite 686; XP002000762**
- **ANGEWANDTE CHEMIE, Bd. 93, Nr. 3, 1981, Seiten 298-299, XP002000760 R. GOMPPER ET AL.: "Aminopyridine, Aminopyrimidine ..."**
- **TETRAHEDRON, Bd. 49, Nr. 45, 1993, Seiten 10205-10218, XP002000761 J. T. GUPTON ET AL.: "The Preparation and Some Reactions ..."**
- **JOURNAL OF HETEROCYCLIC CHEMISTRY, Bd. 28, Nr. 5, 1991, Seiten 1281-1285, XP000563722 J. T. GUPTON ET AL.: "An Alternative Preparation of the ..."**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Es besteht ein hoher industrieller Bedarf an großflächigen Festkörper-Lichtquellen für eine Reihe von Anwendungen, überwiegend im Bereich von Anzeigeelementen, der Bildschirmtechnologie und der Beleuchtungstechnik. Die an diese Lichtquellen gestellten Anforderungen können zur Zeit von keiner der bestehenden Technologien völlig befriedigend gelöst werden.

Als Alternative zu herkömmlichen Beleuchtungs- und Anzeigeelementen, wie Glühlampen, Gasentladungslampen und nicht selbstleuchtenden Flüssigkristallanzeigeelementen, sind bereits seit einiger Zeit Elektrolumineszenz (EL)materialien und -vorrichtungen wie lichtemittierende Dioden (LED) bekannt.

Elektrolumineszenzmaterialien sind Stoffe, die befähigt sind, beim Anlegen eines elektrischen Feldes Licht abzustrahlen. Das physikalische Modell zur Beschreibung dieses Effektes basiert auf der strahlenden Rekombination von Elektronen und Elektronenlücken ("Löchern"). Bei lichtemittierenden Dioden werden die Ladungsträger über die Kathode bzw. Anode in das Elektrolumineszenzmaterial injiziert.
Elektrolumineszenzvorrichtungen enthalten ein Lumineszenzmaterial als lichtemitterende Schicht.
Allgemein sind Elektrolumineszenzmaterialien und -vorrichtungen beispielsweisein Ullmann's Encyclopedia of Industrial Chemistry, Vol A9, 5th Ed. VCH Verlag 1987 und der dort zitierten Literatur sowie in EP-A-0 387 715 und EP-A-0 373 582 beschrieben
Neben anorganischen Stoffen, wie ZnS/Mn oder GaAs, sind auch organische Verbindungen als EL-Materialien bekannt geworden.

Eine Beschreibung von EL-Vorrichtungen, die niedermolekulare organische EL-Materialien enthalten, findet sich beispielsweise in US 4,539,507.

Nachteile dieser niedermolekularen organischen Materialien sind beispielsweise die ungenügenden Filmbildungseigenschaften und eine ausgeprägte Tendenz zur Kristallisation.

In jüngerer Zeit sind auch Polymere als EL-Materialien beschrieben worden (siehe z.B. WO-A 90/13148). Jedoch ist die Lichtausbeute (Quanteneffizienz) bei diesen Stoffen beträchtlich geringer als bei den niedermolekularen Verbindungen.

Aus WO-A-93/06192, EP-A-0 160 790 und EP-A-0 186 045 sind Verbindungen, die zwei Pyrimidingruppen enthalten bekannt.

Wünschenswert war es, EL-Materialien zu finden, die gute Lichtausbeuten zeigen und gleichzeitig zu dünnen homogenen Filmen mit geringer Kristallisationsneigung verarbeitbar sind.

Es wurde nun überraschend gefunden, daß sich konjugierte Verbindungen, welche mindestens zwei Pyrimidinringe enthalten, in hervorragender Weise als Elektrolumineszenzmaterialien eignen.

Gegenstand der Erfindung ist daher die Verwendung von konjugierten Verbindungen, gemäß Anspruch 1, als Elektrolumineszenzmaterialien.

Die Pyrimidinringe enthaltenden konjugierten Verbindungen zeichnen sich durch hohe thermische Stabilität und eine hohe Photostabilität aus. Sie sind teilweise löslich in Säuren, womit neben Aufdampfen auch eine Verarbeitung aus Lösung möglich ist.

Elektrolumineszenzvorrichtungen, die erfindungsgemäße Pyrimidinverbindungen enthalten, zeichnen sich unter anderem durch eine hohe Farbreinheit aus. Mit den erfindungsgemäß verwendeten Verbindungen läßt sich insbesondere auch blaue Elektrolumineszenz erzielen.

Konjugierte Verbindungen, welche zwei oder mehr Pyrimidinringe als Teil des konjugierten Systems enthalten, sind solche der allgemeinen Formel (I),

$$R^1\text{-}F^1_a\text{-}D^1_b\text{-}B^1_c\text{-}A_d\text{-}C^1_e\text{-}E_f\text{-}G_g\text{-}R^2 \quad (I)$$

wobei die Symbole und Indizes folgende Bedeutungen haben:

A ist

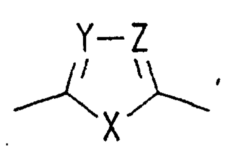

Biphenyl-4,4'-diyl, Anthracen-9,10-diyl, Pyrimidin-2,5-diyl, 1,4-Phenylen;

X ist -O-, -CH = CH-, -CH = N-, -N = CH-;

Y, Z sind gleich oder verschieden $-CR^3 =$, -N =;

$B^1$, $C^1$ sind gleich oder verschieden Pyrimidin-2,5-diyl, 1,4-Phenylen, wobei ein oder zwei Wasserstoffatome durch Reste $R^3$ substituiert sein können, Pyridin-2,5-diyl, Pyridinium-1,4-diyl, Pyridinium-2,5-diyl, wobei der Stickstoff H, eine Alkylgruppe mit 1 bis 20 Kohlenstoffatomen oder eine unsubstituierte Phenylgruppe tragen kann, $-CR^4 = CR^5-$, -C ≡ C-;

$D^1$, E sind gleich oder verschieden A, $B^1$, $C^1$, $-B^1-A-C^1-$;

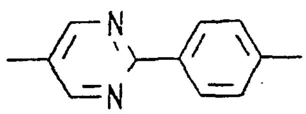 , 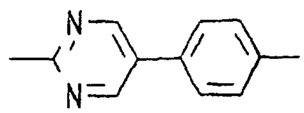 , 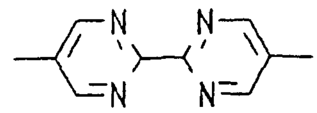 ,

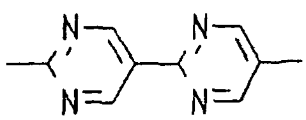 , 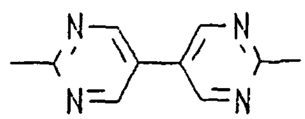 ;

$F^1$, G sind gleich oder verschieden $D^1$, E, Pyridin-2,5-diyl, Pyridinium-1,4-diyl, Pyridinium-2,5-diyl, wobei der Stickstoff H, eine Alkylgruppe mit 1 bis 20 Kohlenstoffatomen oder eine unsubstituierte Phenylgruppe tragen kann, 4-Pyridyl;

$R^1$,$R^2$,$R^3$,$R^4$,$R^5$ sind gleich oder verschieden H, eine unverzweigte oder verzweigte Alkylgruppe mit 1 bis 12 Kohlenstoffatomen, wobei eine oder mehrere $-CH_2$-Gruppen durch -O- ersetzt sein können, - CN, $-NR_2^6$;

$R^6$ ist H, eine Alkylgruppe mit 1 bis 6 Kohlenstoffatomen, Phenyl;

a, c, d, e, g sind gleich oder verschieden 0 oder 1;

b, f sind gleich oder verschieden 0, 1 oder 2;

wobei die Summe a + b + c + d + e + f + g mindestens 3 sein muß und die Gruppe $-F^1_a-D^1_b-B^1_c-A_d-C^1_e-E_f-G_g-$mindestens 2 Pyrimidin-2,5-diyl-Gruppen enthalten muß.

Bevorzugt sind Verbindungen der Formel (I) in denen die Symbole und Indizes folgende Bedeutungen haben:

A ist Pyrimidin-2,5-diyl, 1,4-Phenylen;

$B^1$, $C^1$ sind gleich oder verschieden Pyrimidin-2,5-diyl, 1,4-Phenylen, wobei ein oder zwei Wasserstoffatome durch Reste $R^3$ substituiert sein können, -CH = CH-;

$D^1$, E sind gleich oder verschieden A, $B^1$, $C^1$, $-B^1-A-C^1-$,

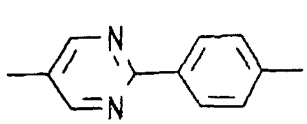 , 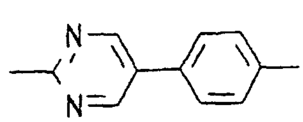 , 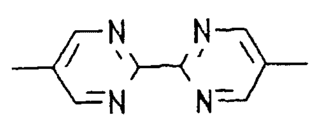 ,

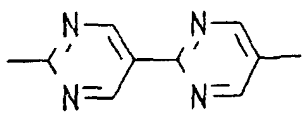 , 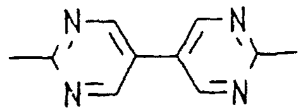 ;

| | |
|---|---|
| $F^1$, G | sind gleich oder verschieden $D^1$, E; |
| $R^1$,$R^2$,$R^3$ | sind gleich oder verschieden H, eine unverzweigte oder verzweigte Alkylgruppe mit 1 bis 12 Kohlenstoffatomen, wobei eine $-CH_2$-Gruppe durch -O- ersetzt sein kann, -CN, $-NR_2^6$; |
| $R^6$ | ist H, eine Alkylgruppe mit 1 bis 6 Kohlenstoffatomen, Phenyl; |
| a, c, d, e, g | sind gleich oder verschieden 0 oder 1; |
| b. f | sind gleich oder verschieden 0. 1 oder 2: |

wobei die Summe a + b + c + d + e + f + g mindestens 3 sein muß und die Gruppe $-F^1_a-D^1_b-B^1_c-A_d-C^1_e-E_f-G_g-$ mindestens 2 Pyrimidin-2,5-diyl-Gruppen enthalten muß.

Weiterhin bevorzugt ist die Verwendung von Verbindungen der Formel (I), in denen die Summe a + b + c + d + e + f + g mindestens 4, vorzugsweise 4 bis 16, besonders bevorzugt 5 bis 16, ganz besonders bevorzugt 5 bis 12 ist.

Besonders bevorzugt sind Verbindungen der Formel (I), in denen die Symbole und Indizes folgende Bedeutungen haben:

| | |
|---|---|
| A | ist Pyrimidin-2,5-diyl, 1,4-Phenylen; |
| $B^1$, $C^1$ | sind gleich oder verschieden Pyrimidin-2,5-diyl, 1,4-Phenylen, -CH = CH-; |
| $D^1$, E | sind gleich oder verschieden A, $B^1$, $C^1$, $-B^1-A-C^1-$, |

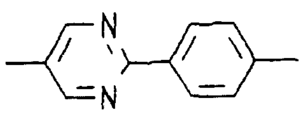 , 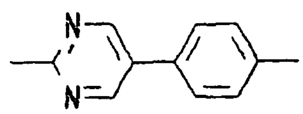 , 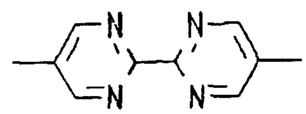 ,

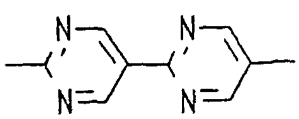 , 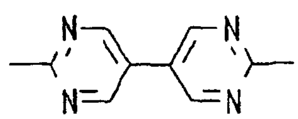 ;

| | |
|---|---|
| $F^1$, G | sind gleich oder verschieden $D^1$, $E^1$; |
| $R^1$,$R^2$ | sind H, eine unverzweigte oder verzweigte Alkylgruppe mit 1 bis 12 Kohlenstoffatomen, $-NR_2^6$; |
| $R^6$ | ist H, eine Alkylgruppe mit 1 bis 6 Kohlenstoffatomen, Phenyl; |
| a, c, d, e, g | sind gleich oder verschieden 0 oder 1; |
| b, f | sind gleich oder verschieden 0, 1 oder 2; |

wobei die Summe a + b + c + d + e + f + g mindestens 3 sein muß und die Gruppe $-F^1_a-D^1_b-B^1_c-A_d-C^1_e-E_f-G_g-$ mindestens 2 Pyrimidin-2,5-diyl-Gruppen enthalten muß.

Ganz besonders bevorzugt sind Verbindungen der allgemeinen Formel (I), in denen die Gruppe $-F^1_a-D^1_b-B^1_c-A_d-C^1_e-E_f-G_g-$ ausgewählt ist aus der Gruppe:

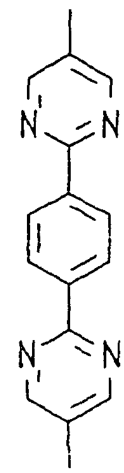

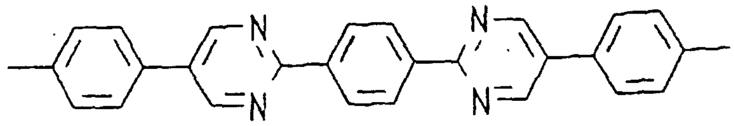

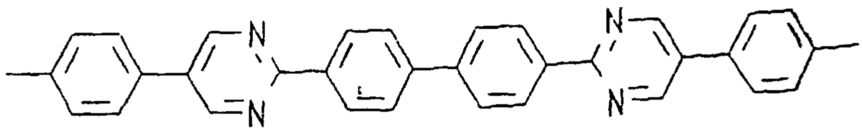

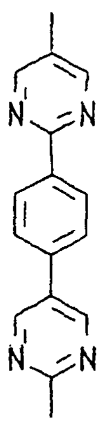

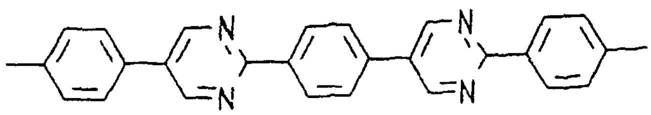

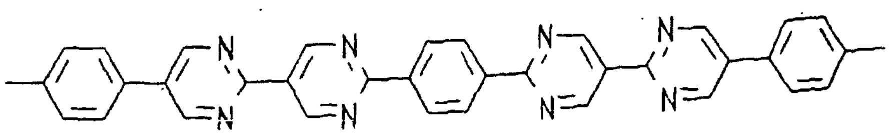

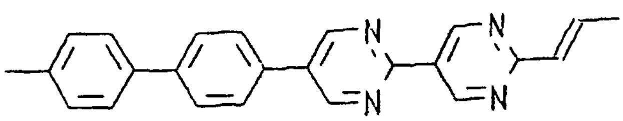

,

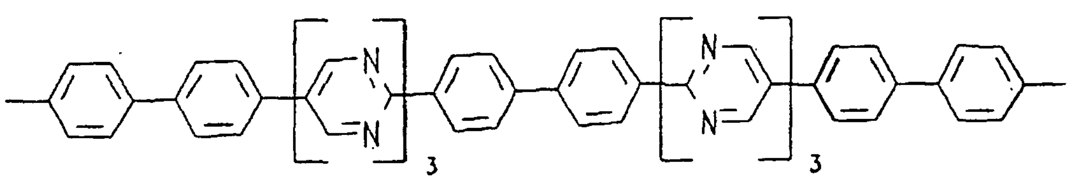

,

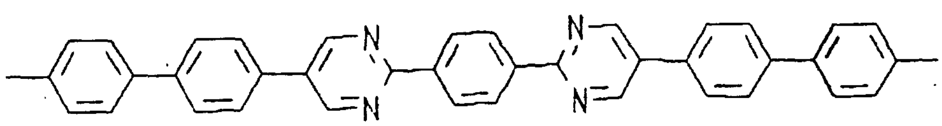

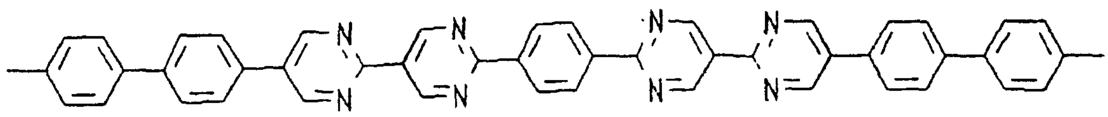

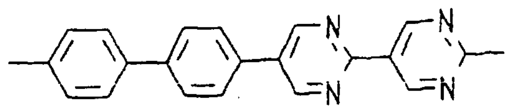

und $R^1$ und $R^2$ die in der Formel (I) angegebenen Bedeutungen haben.

Die Verbindungen der Formel (I) sind teilweise bekannt und teilweise neu.

Gegenstand der Erfindung sind daher auch Pyrimidinverbindungen der allgemeinen Formel (I), gemäß Anspruch 7.

Bevorzugte und besonders bevorzugte Bedeutungen für die Symbole $R^1$ bis $R^6$, A, $B^1$, $C^1$, $D^1$, E, $F^1$, G, X, Y, Z in der Formel (I) der erfindungsgemäßen Verbindungen sind die obengenannten.

Insbesondere bevorzugte Pyrimidinverbindungen der Formel (I) sind solche, in denen die Gruppe $-F^1_a-D^1_b-B^1_c-A_d-C^1_e-E_f-G_g-$ ausgewählt ist aus der Gruppe:

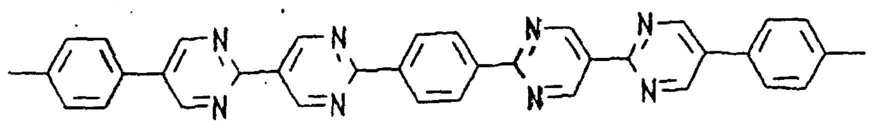

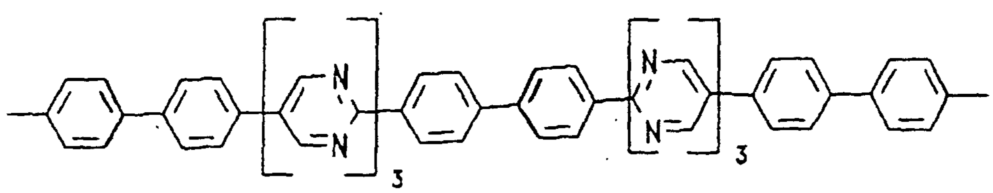

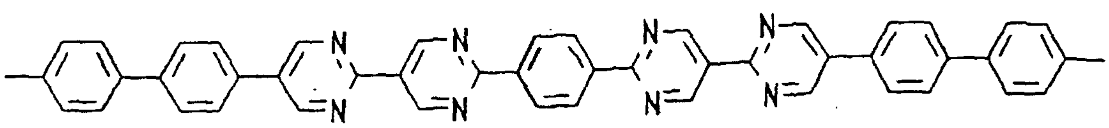

Die Herstellung erfindungsgemäßer oder erfindungsgemäß verwendeter Verbindungen der Formel (I) kann nach an sich literaturbekannten Methoden erfolgen, wie sie in Standardwerken zur Organischen Synthese, z.B. Houben-Weyl, Methoden der Organischen Chemie, Georg-Thieme-Verlag, Stuttgart, beschrieben werden.

Die Herstellung erfolgt dabei unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, und zwar derart, daß man sie aus dem Reaktionsgemisch nicht isoliert, sondern sofort weiter zu den Verbindungen der Formel (I) umsetzt.

Beispielsweise sei verwiesen auf DE-A 23 44 732, 24 50 088, 24 29 093, 26 36 684, 27 01 591 und 27 52 975 für Verbindungen mit 1,4-Phenylen-Gruppen; DE-A 26 41 724 für Verbindungen mit Pyrimidin-2,5-diyl-Gruppen; DE-A 40 26 223 und EP-A 0 391 203 für Verbindungen mit Pyridin-2,5-diyl-Gruppen.

Die Herstellung disubstituierter Pyridine und disubstituierter Pyrimidine findet sich beispielsweise auch in den entsprechenden Bänden der Serie "The Chemistry of Heterocyclic Compounds" von A. Weissberger und E.C. Taylor (Herausgeber).

Was die Verknüpfung der Ringsysteme miteinander angeht, sei beispielsweise verwiesen auf:
N. Miyaura, T. Yanagai und A. Suzuki in Synthetic Communications 11 (1981) 513-519, DE-C-39 30 663, M.J. Sharp, W. Cheng, V. Snieckus in Tetrahedron Letters 28 (1987) 5093; G.W. Gray in J. Chem. Soc. Perkin Trans 111989, 2041 und Mol. Cryst. Liq. Cryst. 172 (1989) 165; 204 (1991) 43 und 91; EP-A 0 449 015; WO-A 89/12039; WO-A 89/03821; EP-A 0 354 434 für die direkte Verknüpfung von Aromaten und Heteroaromaten und Koji Seto et al. in Liquid Crystals 8 (1990) 861 für Verbindungen mit -C ≡ C-Brückengliedern.

Die Herstellung von Verbindungen der Formel (I) kann beispielsweise auch durch Umsetzung von substituierten Arylvinamidiniumsalzen mit Amidinen erfolgen.

Unter Zugrundelegung dieses Pyrimidin-Syntheseprinzips kann man durch wiederholte Kondensation von Vinamidiniumsalzen mit Amidinen und nachfolgend erneuter Vinamidinierung definierte p-Oligopyrimidine aufbauen (Schema 1).

Schema 1

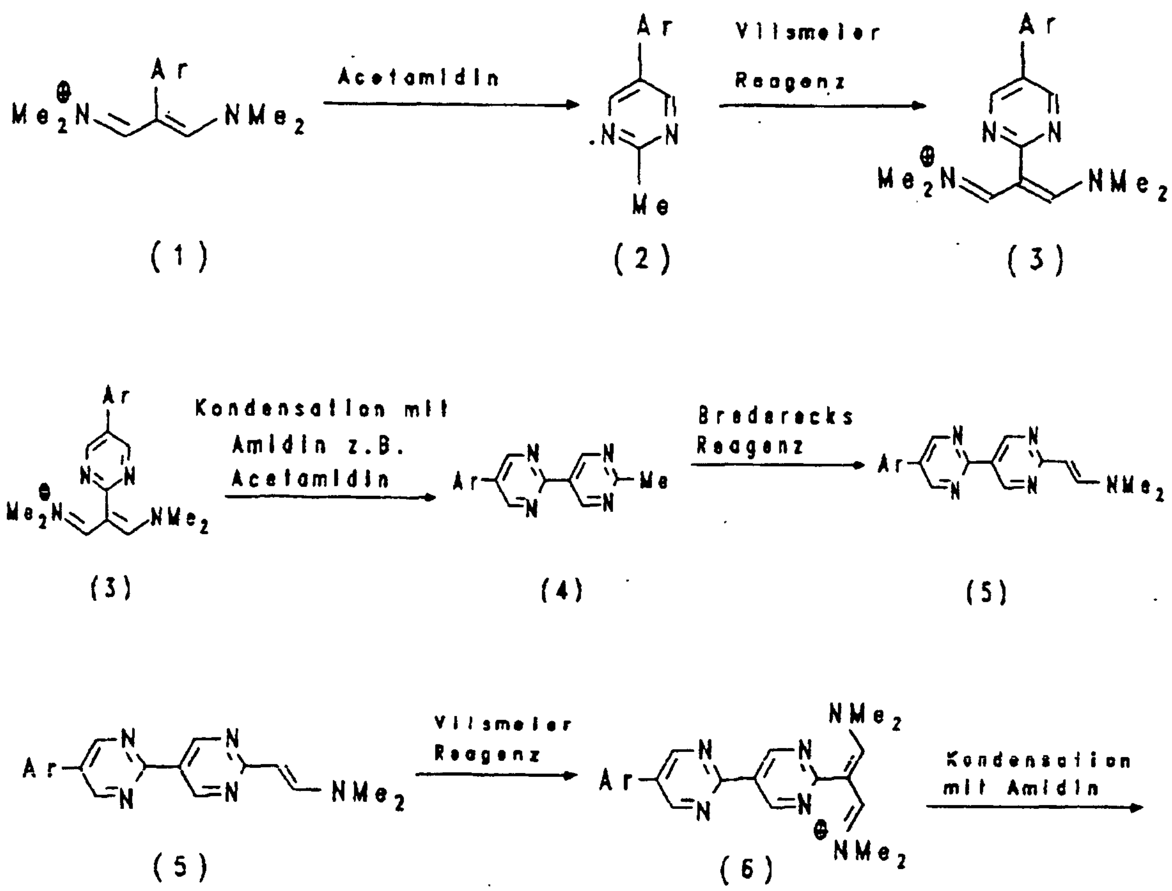


Die Herstellung der Heteroaryl-Vinamidiniumsalze (3) erfolgt beispielsweise durch Umsetzung von methylsubstituierten Heteroaromaten (2) unter den Bedingungen der Vilsmeier-Haack Reaktion (siehe z.B. de Meheas, Bull. Soc. Chim. Fr. (1962) 1989-1999), beispielsweise mit Dimethylformamid (DMF) und Oxalylchlorid.

Methylsubstituierte Heteroaromaten (2) können nach bekannten Methoden aus arylsubstituierten Vinamidiniumsalzen (1) durch Kondensation mit geeigneten Stickstoffverbindungen, wie Acetamidin, hergestellt werden. Analog können die Vinamidiniumsalze (3) zu den methylsubstituierten Verbindungen (4) umgesetzt werden.

Beim weiteren Aufbau von Verbindungen der Formel (I) ist es aus Gründen der Ausbeute und der Reinheit der erhaltenen Produkte vorteilhaft, den methylsubstituierten Heteroaromaten (4) zunächst durch Umsetzung mit beispielsweise dem Brederecks-Reagenz (t-Butyloxybis(dimethylamino)methan) in das Enamin (5) überzuführen und daraus anschließend unter Vilsmeier-Haack-Bedingungen das Vinamidiniumsalz (6) herzustellen.

Das Vinamidiniumsalz (6) kann nach literaturbekannten Methoden zu höheren Oligomeren der Formel (I) kondensiert werden.

In den oben aufgeführten Reaktionen dienen im allgemeinen komplexe Anionen, wie $PF_6^{\ominus}$, $BF_4^{\ominus}$ oder $ClO_4^{\ominus}$, als Gegenionen. Bevorzugt ist die Verwendung von $ClO_4^{\ominus}$.

Bringt man Vinamidiniumsalze (7) mit Arylenbis(pertrimethylsilyl)carbamidin (6) im Molverhältnis 2:1, bezogen auf das Vinamidiniumsalz (7), unter Zusatz eines Desilylierungsmittels, wie KF, zur Reaktion, so erhält man infolge zweifacher Pyrimidinbildung symmetrische Pyrimidinverbindungen (Schema 2).

Schema 2

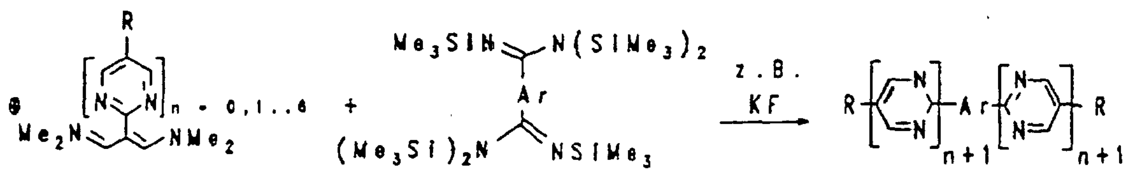


(7) (8) (9)

In Umkehr der Synthesestrategie können auch Arylenbisvinamidiniumsalze (10) mit Amidinen umgesetzt werden. Dadurch gelingt die Herstellung von symmetrischen Pyrimidinverbindungen (11) mit gegenüber dem vorigen Beispiel umgekehrter Pyrimidinpolarität, wobei (11) analog dem obengenannten Reaktionsschema 1 über das Bisenamin (12) und das Bisvinamidiniumsalz (13) weiter zu definierten höheren Oligomeren der Formel (I) umgesetzt werden kann (Schema 3).

Schema 3

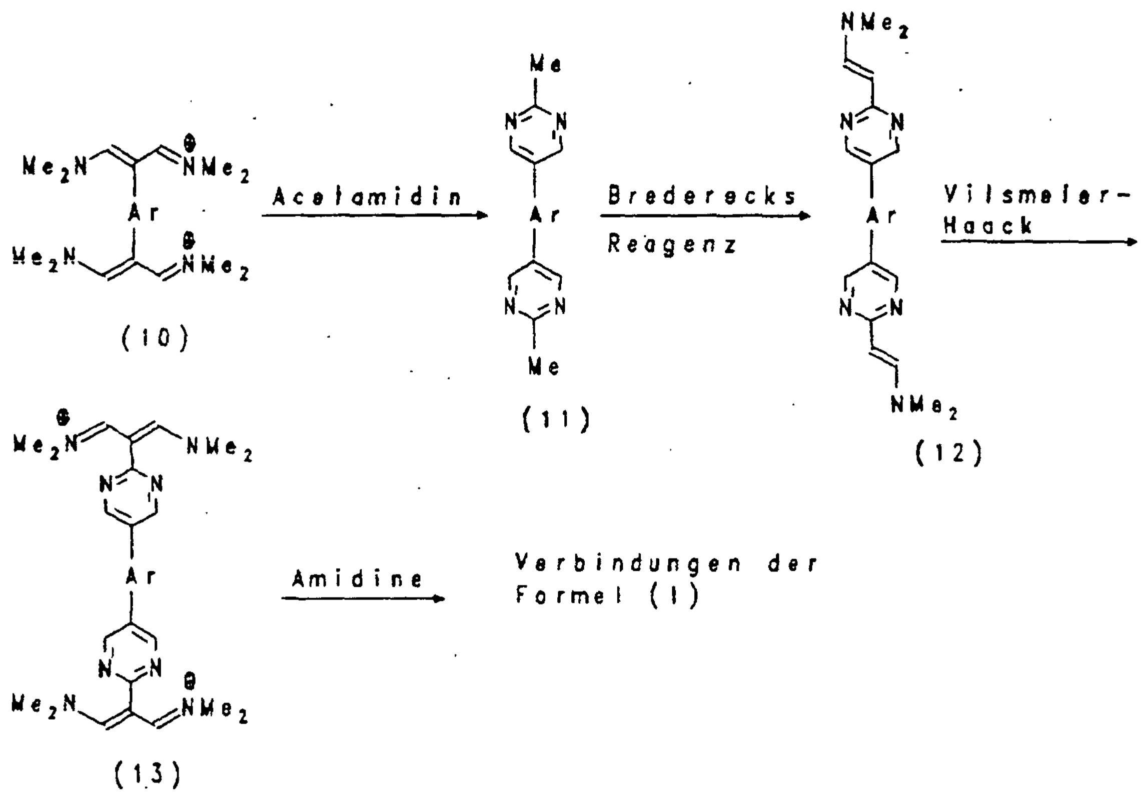


(10) (11) (12) (13)

Die Herstellung der Arylgruppen (Ar in Schema 1-3) kann dabei nach an sich literaturbekannten Methoden erfolgen,

wie sie in Standardwerken zur Organischen Synthese, z.B. Houben-Weyl, Methoden der Organischen Chemie, Georg-Thieme-Verlag, Stuttgart und in den entsprechenden Bänden der Serie "The Chemistry of Heterocyclic Compounds" von A. Weissberger und E.C. Taylor (Herausgeber) beschrieben werden.

Gegenstand der Erfindung ist daher auch ein Verfahren zur Herstellung von 2,5'-Bipyrimidinderivaten, dadurch gekennzeichnet, daß ein 2-Methylpyrimidinderivat mit einem Enaminierungsreagenz in das entsprechende Enamin übergeführt wird, anschließend mit einem Vilsmeier-Haack-Reagenz zum Vinamidiniumsalz umgesetzt wird, dieses gegebenenfalls mit einem komplexen Anion gefällt wird und das so erhaltene Vinamidiniumsalz mit einem Amidin umgesetzt wird.

Das Verfahren liefert wohldefinierte Produkte in guten Ausbeuten und hoher Reinheit. Es ist in hervorragender Weise zum Aufbau von 2,5'; 2',2"-Terpyrimidinverbindungen und ganz allgemein höheren 2,5-verknüpften Oligopyrimidinen geeignet.

Die verwendeten Enaminierungsreagenzien sind aus der einschlägigen Literatur bekannt, bevorzugt sind t-Butyloxybis(dimethylamino)methan (Brederecks-Reagenz), DMF/Dimethylsulfat oder Dimethylformamidacetale, besonders bevorzugt ist das Brederecks-Reagenz.

Vorzugsweise dient das Enaminierungsreagenz auch als Lösungsmittel; es können aber auch Gemische aus dem Enaminierungsreagenz und beispielsweise Pyridin, DMSO, DMF, N-Methylpyrrolidon, vorzugsweise Pyridin, verwendet werden.

Das Enaminierungsreagenz kann äquimolar oder im Überschuß eingesetzt werden; dient es als Lösungsmittel, ist ein 1 bis 100facher, insbesondere 1 bis 5facher, Überschuß bevorzugt.

Der Enaminierungsschritt läuft im allgemeinen bei einer Temperatur von 100° bis 200°C, vorzugsweise 140 bis 160°C, ab.

Das Enamin kann nach literaturbekannten Methoden, wie unter anderem in den Beispielen angegeben, aufgearbeitet werden.

Für Umsetzung zum Vinamidiniumsalz kommen grundsätzlich alle gängigen Vilsmeier-Reagenzien in Frage, wie $POCl_3$, Oxalylchlorid, $COCl_2$, $O(SO_2\text{-}CF_3)_2$, bevorzugt ist die Verwendung von Oxalylchlorid.

Die Reaktion wird üblicherweise in DMF oder in Gemischen aus DMF und anderen geeigneten Lösungsmitteln, wie Methylenchlorid, Chloroform, oder auch in einem Mehrphasensystem aus DMF und damit nicht mischbaren Lösungsmitteln, wie Nitrobenzol, durchgeführt.

Die Reaktion wird im allgemeinen bei einer Temperatur von -60 bis + 100°C, vorzugsweise -50 bis + 70°C, durchgeführt.

Man setzt Enamin und Vilsmeier-Reagenz üblicherweise im Verhältnis 1:1-3, vorzugsweise 1:1-2 ein.

Die Aufarbeitung erfolgt nach gängigen, teilweise in den Beispielen beschriebenen Methoden.

Wohldefinierte Vinamidiniumsalze lassen sich zweckmäßigerweise durch Fällung mit einem komplexen Anion, wie $BF_4^{\ominus}$, $PF_6^{\ominus}$, $ClO_4^{\ominus}$, Tetracyanopropenid, vorzugsweise $ClO_4^{\ominus}$, herstellen.

Die Fällung erfolgt im allgemeinen aus Wasser, wobei das Fällungsmittel vorzugsweise in bis zu 10fachem Überschuß zugesetzt wird.

Das so erhaltene Vinamidiniumsalz wird weiter mit an sich bekannten Amidinen umgesetzt.

Im allgemeinen beträgt das Molverhältnis Vinamidiniumsalz zu Amidin 0,2-5:1, vorzugsweise 0,5-1:1.

Das Vinamidiniumsalz und ein Amidin werden in einem Lösungsmittel in Gegenwart einer Base zur Reaktion gebracht, wobei Lösungsmittel und Base identisch sein können.

Beispiele für geeignete Lösungsmittel sind Pyridin, Alkohole, wie Methanol, Ethanol, Wasser oder auch Gemische wie Pyridin/Eisessig.

Geeignete Basen sind beispielsweise Pyridin, Carbonate, wie $K_2CO_3$, oder Alkoholate, wie Methanolat.

Die Reaktion wird üblicherweise bei einer Temperatur von 60 bis 180°C, vorzugsweise 80 bis 120°C, durchgeführt.

Die Aufarbeitung der erfindungsgemäß hergestellten 2,5'-Bipyrimidinderivate erfolgt nach an sich bekannten, dem Fachmann geläufigen Methoden, wie sie unter anderem in den Beispielen beschrieben sind.

Die Herstellung von Bis- oder Oligopyrimidinverbindungen, kann durch Umsetzung eines Vinamidiniumsalzes in Gegenwart eines Desilylierungsmittels in einem organischen Lösungsmittel bei einer Temperatur von 0 bis 250°C mit einer (Hetero)-Arylenbis(pertrimethylsilyl)carbamidinverbindung erfolgen.

Ausgangsverbindungen dieses Verfahrens sind Vinamidiniumsalze der Formel (II),

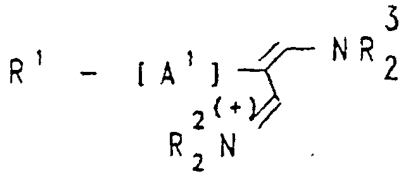 (II)

und (Hetero)-Arylenbis(pertrialkylsilyl)carbamidinverbindungen der Formel (III),

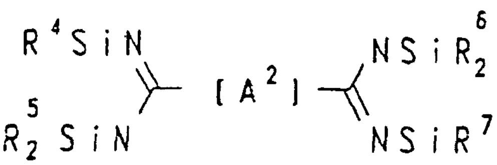 (III)

wobei die Symbole mit Indizes folgende Bedeutungen haben:

| | |
|---|---|
| $A^1$,$A^2$ | sind gleich oder verschieden 1,4-Phenylen, Pyrazin-2,5-diyl, Pyridazin-3,6-diyl, Pyridin-2,5-diyl, Pyrimidin-2,5-diyl, 1,3,4-Oxadiazol-2,5-diyl, 1,3-Oxadiazol-2,4-diyl, 1,3-Oxadiazol-2,5-diyl, 1,3,4-Triazol-2,5-diyl, 1,3,4-Thiadiazol-2,5-diyl, 1,3-Thiazol-2,4-diyl, 1,3-Thiazol-2,5-diyl, Thiophen-2,4-diyl, Thiophen-2,5-diyl, Pyrrol-2,5-diyl, Pyrrol-2,4-diyl, Furan-2,5-diyl, Furan-2,4-diyl, Naphthalin-2,6-diyl; |
| $R^1$,$R^2$,$R^3$,$R^4$,$R^5$ | sind gleich oder verschieden H, eine unverzweigte oder verzweigte Alkylgruppe mit 1 bis 12 Kohlenstoffatomen, wobei eine oder mehrere -$CH_2$-Gruppen durch -O- ersetzt sein können, - CN, $NR_2^6$; |
| $R^6$ | ist H, eine Alkylgruppe mit 1 bis 6 Kohlenstoffatomen, Phenyl. |

Es können alle denkbaren Desilylierungsmittel, wie $TiCl_3$ oder Basen, eingesetzt werden. Bevorzugte Desilylierungsmittel sind jedoch Fluoridionen, die in Form eines anorganischen oder organischen Fluorids, wie KF, $NH_4F$, $Bu_4NF$, vorzugsweise KF, zugesetzt werden.
Das Desilylierungsmittel wird im allgemeinen stöchiometrisch oder bis zum 5fachen Überschuß, vorzugsweise stöchiometrisch, in Bezug auf die vorhandenen Silylgruppen eingesetzt.
Geeignete Lösungsmittel für das Verfahren sind beispielsweise Dimethylformamid, Pyridin, DMSO, N-Methylpyrrolidon, Alkohole oder Gemische dieser Lösungsmittel
. Bevorzugt ist die Verwendung einer Mischung von Pyridin/DMF, vorzugsweise im Verhältnis 3/1.
Das Verfahren wird zweckmäßigerweise bei einer Temperatur von 0 bis 200°C, vorzugsweise 90 bis 150°C, durchgeführt.
Die Reaktionsdauer beträgt im allgemeinen 3 bis 15 h.
Die Aufarbeitung kann nach allgemein bekannten, dem Fachmann geläufigen Methoden erfolgen, wie sie unter anderem in den Beispielen aufgeführt sind.
Erfindungsgemäß finden die beschriebenen Pyrimidinverbindungen der Formel (I) als Eieldrolumineszenzmaterialien Verwendung; d.h., sie dienen als aktive Schicht in einer Elektrolumineszenzvorrichtung. Als aktive Schicht im Sinne der Erfindung gelten Elektrolumineszenzmaterialien, die befähigt sind bei Anlegen eines elektrischen Feldes Licht abzustrahlen (lichtemittierende Schicht), sowie Materialien, welche die Injektion und/oder den Transport der positiven und/oder negativen Ladungen verbessern (Ladungsinjektionsschichten und Ladungstransportschichten)
Gegenstand der Erfindung ist daher auch eine Elektrolumineszenzvorrichtung gemäß Anspruch 6 mit einer oder mehreren aktiven Schichten, die eine oder mehrere Verbindungen der Formel (I) enthält. Die aktive Schicht kann beispielsweise eine lichtemittierende Schicht und/oder eine Transportschicht und/oder ein Ladungsinjektionsschicht sein.
Der allgemeine Aufbau solcher Elektrolumineszenzvorrichtungen ist beispielsweise in US 4,539,507 und US 5,151,629 beschrieben.
Sie enthalten üblicherweise eine elektrolumineszierende Schicht zwischen einer Kathode und einer Anode, wobei mindestens eine der Elektroden transparent ist. Zusätzlich kann zwischen der elektrolumineszierenden Schicht und der Kathode eine Elektroneninjektions und/oder Elektronentransportschicht eingebracht sein und/oder zwischen der elektrolumineszierenden Schicht und der Anode eine Lochinjektions und/oder Lochtransportschicht eingebracht

sein. Als Kathode können z.B. Ca, Mg, Al, In, Mg/Ag dienen. Als Anode können z.B. Au oder ITO (Indiumoxid/Zinnoxid auf einem transparentem Substrat, z.B. aus Glas oder einem transparenten Polymer) dienen.

Im Betrieb wird die Kathode auf negatives Potential gegenüber der Anode gesetzt, dabei werden Elektronen von der Kathode in die Elektroneninjektionsschicht /Elektronentransportschicht oder direkt in die lichtemittierende Schicht injiziert. Gleichzeitig werden Löcher von der Anode in die Lochinjektionsschicht/ Lochtransportschicht oder direkt in die lichtemittierende Schicht injiziert.

Die injizierten Ladungsträger bewegen sich unter dem Einfluß der angelegten Spannung durch die aktiven Schichten aufeinander zu. Dies führt an der Grenzfläche zwischen Ladungstransportschicht und lichtemittierender Schicht oder innerhalb der lichtemittierenden Schicht zu Elektronen/Loch-Paaren, die unter Aussendung von Licht rekombinieren. Die Farbe des emittierten Lichtes kann durch die als lichtemittierende Schicht verwendete Verbindung variiert werden.

Elektrolumineszenzvorrichtungen finden Anwendung z.B. als selbstleuchtende Anzeigeelemente, wie Kontrollampen, alphanumerische Displays, Hinweisschilder, und in optoelektronischen Kopplern.

Die Erfindung wird durch die Beispiele näher erläutert, ohne sie darauf beschränken zu wollen.

Beispiel 1

2-[2'-(5'-Phenyl)-pyrimidinyl]-3-dimethylamino-N,N-dimethyl-prop-2-eniminiumperchlorat (14)

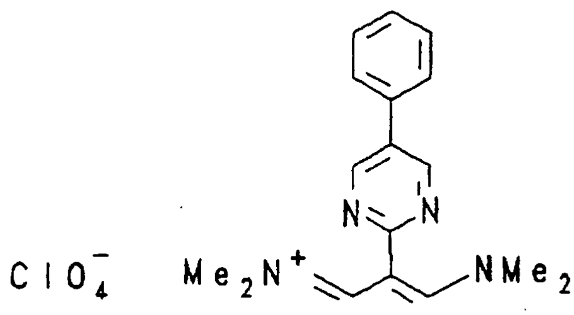

(14)

Zu 6.82 ml (88.13 mmol, 30 Äquiv.) wasserfreiem DMF wurden bei - 50°C unter heftigem Rühren 0.63 ml (7.34 mmol, 2,5 Äquiv.) Oxalylchlorid getropft. Nach 20 min Auftauen wurden zu dem Reaktionsgemisch 0.50 g (2.94 mmol) 2-Methyl-5-phenyl-pyrimidin gegeben (siehe R. M. Wagner, C. Jutz, Chem. Ber. 104 (1971) 2975) und die Suspension wurde 18 h auf 45°C erwärmt. Das Reaktionsgemisch wurde nach dem Abkühlen mit 10 ml Wasser versetzt, die erhaltene klare Lösung anschließend bei Raumtemperatur langsam zu 4.13 g (29.38 mmol, 10 Äquiv.) Natriumperchlorat-Monohydrat, gelöst in 200 ml Wasser, getropft. Der dabei entstandene Niederschlag wurde abgesaugt und mit viel Wasser gewaschen. Ausb. 0.94 g (84 %) beiges mikrokristallines Pulver. Für die meisten Umsetzungen erwies sich die Reinheit des Rohproduktes als ausreichend; für analytische Zwecke wurden 0.30 g des Rohproduktes aus 50 ml Acetonitril umkristallisiert. Ausb. 0.28 g (71 %); farblose Kristalle.

UV/VIS (DMSO): $\lambda_{max}$ (lg $\varepsilon$) = 313 nm (4.455).

$^1$H-NMR ([D]TFA): $\delta$ = 3.45 (s, 6H, 2 $NCH_3$), 3.76 (s, 6H, 2 $NCH_3$), 7.60 - 7.66 (m, 3H, Ph-3-H, Ph-4-H, Ph-5-H), 7.68 - 7.73 (m, 2H, Ph-2-H, Ph-6H), 8.40 (s, 2H, 1-H, 3-H), 9.29 (s, 2H, Pym).

| | | | | |
|---|---|---|---|---|
| $C_{17}H_{21}ClN_4O_4$ (380.8) | Ber. | C 53.62 | H 5.56 | N 14.71 |
| | Gef. | C 53.63 | H 5.55 | N 14.48 |

Beispiel 2

2,2'-(p-Phenylen)-di-pyrimidin (15)

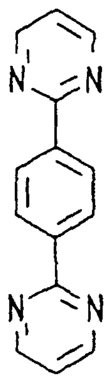

(15)

Eine Suspension von 1.50 g (6.62 mmol) 3-Dimethylamino-N,N-dimethyl-prop-2-eniminium-perchlorat (S. S. Malhotra, M. C. Whiting J. Chem. Soc. (1960) 3812) und 1.97 g (3.31 mmol, 0.5 Äquiv.) Phenylen-1,4-bis(pertrimethylsilylcarbamidin) (R. T. Boere, R. T. Oakley, R. W. Reed, J. Organomet. Chem. 331 (1987) 161) in 35 ml Pyridin wurde unter Zusatz von 1.15 g (19.85 mmol, 3 Äquiv.) Kaliumfluorid 6 h unter Rückfluß zum Sieden erhitzt. Der teilweise schon während des Erhitzens ausgefallene Niederschlag wurde nach dem Abkühlen abgesaugt, mit viel Wasser und anschließend mit Methanol gewaschen. Die Umkristallisation des farblosen Rohprodukts erfolgte aus 120 ml DMSO. Ausb. 0.64 g (81 %), farblose Plättchen, Schmp. > 330°C.
UV/VIS (DMSO): $\lambda_{max}$ (lg ε) = 294 nm (4.308).
Fluoreszenz (DMSO): $\lambda_{max}$ = 367 nm
$^1$H-NMR ([D]TFA): δ = 8.20 (t, J = 6 Hz, 2H, Pym-5-H), 8.74 (s, 4H, Phn), 9.50 (d, J = 6 Hz, 4H, Pym-4-H, Pym-6-H).

| | | | | |
|---|---|---|---|---|
| $C_{14}H_{10}N_4 \cdot 1/3H_2O$ (240.3) | Ber. | C 69.99 | H 4.44 | N 23.32 |
| | Gef. | C 69.99 | H 4.37 | N 23.09 |

Beispiel 3

5,5'-Bis-(phenyl)-2,2'-(p-phenylen)-di-pyrimidin (16)

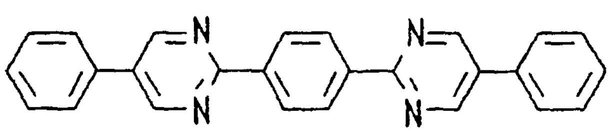

(16)

Eine Suspension von 1.50 g (4.95 mmol) 2-Phenyl-3-dimethylamino-N,N-dimethyl-prop-2-eniminium-perchlorat (S. N. Balasubrahmanyam, A. S. Radhakrishna, J. Chem. Soc., Perkin Trans. 2 (1977) 1388) und 1.47 g (2.48 mmol, 0.5 Äquiv.) Phenylen-1,4-bis(pertrimethylsilylcarbamidin) (R. T. Boere, R. T. Oakley, R. W. Reed, J. Organomet. Chem. 331 (1987) 161) in 50 ml Pyridin wurde unter Zusatz von 0.86 g (14.86 mmol, 3 Äquiv.) Kaliumfluorid 5 h unter Rückfluß zum Sieden erhitzt. Der teilweise schon während des Erhitzens ausgefallene Niederschlag wurde nach dem Abkühlen abgesaugt, mit viel Wasser und anschließend mit Methanol gewaschen. Die Umkristallisation des farblosen Rohprodukts erfolgte aus 150 ml DMSO. Ausb. 0.88 g (92 %), farblose Kristallplättchen, Schmp. > 330°C.

UV/VIS (DMSO): $\lambda_{max}$ (lg ε) = 332 nm (4.342).
Fluoreszenz (DMSO): $\lambda_{max}$ = 398 nm
$^1$H-NMR ([D]TFA): δ = 7.69 - 7.75 (m, 6H, Ph-3-H, Ph-4-H, Ph-5-H), 7.82 - 7.87 (m, 4H, Ph-2-H, Ph-6-H), 8.79 (s, 4H, Phn), 9.67 (s, 4H, Pym).

| | | | | |
|---|---|---|---|---|
| $C_{26}H_{18}N_4$ (386.5) | Ber. | C 80.81 | H 4.69 | N 14.50 |
| | Gef. | C 81.11 | H 4.70 | N 14.22 |

Beispiel 4

5,5'-Bis-(phenyl)-2,2'-(p-biphenylen)-di-pyrimidin (17)

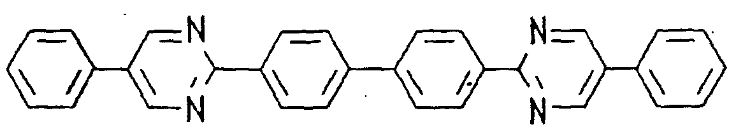

(17)

Eine Suspension von 1.50 g (4.95 mmol) 2-Phenyl-3-dimethylamino-N,N-dimethyl-prop-2-eniminium-perchlorat (siehe S. N. Balasubrahmanyam, A. S. Radhakrishna, J. Chem. Soc.,Perkin Trans. 2 (1977) 1388) und 1.66 g (2.48 mmol, 0.5 Äquiv.) 1,1'-Biphenylen-4,4'-bis(pertrimethylsilylcarbamidin) (Herstellung analog dem Phenylen-1,4-bis(pertrimethylsilylcarbamidin)) in 60 ml Pyridin wurde unter Zusatz von 0.86 g (14.86 mmol, 3 Äquiv.) Kaliumfluorid 5 h unter Rückfluß zum Sieden erhitzt. Der teilweise schon während des Erhitzens ausgefallene Niederschlag wurde nach dem Abkühlen abgesaugt, mit viel Wasser und anschließend mit Aceton gewaschen. Die Umkristallisation des farblosen Rohprodukts erfolgte aus 200 ml DMSO. Ausb. 1.02 g (89 %), farblose Plättchen, Schmp. > 330°C.
UV/VIS (DMSO): $\lambda_{max}$ (lg ε) = 336 nm (4.306).
Fluoreszenz (DMSO): $\lambda_{max}$ = 382, 397 nm
$^1$H-NMR ([D]TFA): δ = 7.66 - 7.73 (m, 6H, Ph-3-H, Ph-4-H, Ph-5-H), 7.78 - 7.84 (m, 4H, Ph-2-H, Ph-6-H), AA'BB'-Signal zentriert bei 8.14 ($^3J$ = 8 Hz, 4H, Biphn-2-H, Biphn-6-H) und 8.59 ($^3J$ = 8 Hz, 4H, Biphn-3-H, Biphn-5-H), 9.59 (s, 4H, Pym).

Beispiel 5

2,2'-Bis-(phenyl)-5,5'-(p-phenylen)-di-pyrimidin (18)

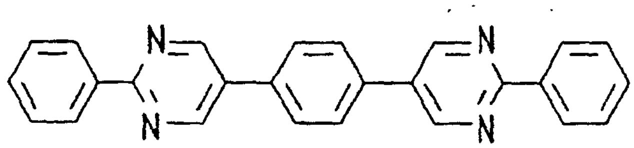

(18)

1.00 g (1.90 mmol) Phenylen-1,4-bis-(3-dimethylamino-N,N-dimethyl-prop-2-eniminium-perchlorat) (Z. Arnold, Collect. Czech. Chem. Commun. 30 (1965) 2783) wurden mit 0.74 g (4.74 mmol, 2.5 Äquiv.) Benzamidinhydrochlorid-Hydrat in 40 ml Pyridin 5 h unter Rückfluß zum Sieden erhitzt. Nach dem Abkühlen wurde der ausgefallene Niederschlag abgesaugt und mit viel Wasser und nachfolgend Methanol gewaschen. Die Umkristallisation des schwach braunen Rohprodukts aus 70 ml DMSO lieferte farblose Plättchen. Ausb. 0.66 g (90 %), Schmp. 299°C.
UV/VIS (DMSO): $\lambda_{max}$ (lg ε) = 321 nm (4.680).
Fluoreszenz (DMSO): $\lambda_{max}$ = 437 nm

$^1$H-NMR ([D]TFA): δ = AA'BB'C-Signal zentriert bei 7.78 ($^3$J = 8 Hz, 4H, Ph-3-H, Ph-5-H), bei 7.91 ($^3$J = 8 Hz, 2H, Ph-4-H) und bei 8.44 ($^3$J = 8 Hz, 4H, Ph-2-H, Ph-6-H), 8.14 (s, 4H, Phn), 9.67 (s, 4H, Pym).

Beispiel 6

5,5'-(p-Phenylen)-di-pyrimidin (19)

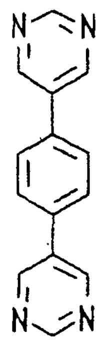

(19)

2.00 g (3.79 mmol) Phenylen-1,4-bis-(3-dimethylamino-N,N-dimethyl-prop-2-eniminium-perchlorat) (Z. Arnold, Collect. Czech. Chem. Commun. 30 (1965) 2783) wurden mit 0.92 g (11.38 mmol, 3 Äquiv.) Formamidin-hydrochlorid in 30 ml Pyridin 12 h unter Rückfluß zum Sieden erhitzt. Nach dem Abkühlen wurde der ausgefallene Niederschlag abgesaugt, mit Wasser und Methanol gewaschen und aus 50 ml DMSO umkristallisiert. Ausb. 0.68 g (77 %), farblose Plättchen, Schmp. 258°C.
UV/VIS (DMSO): $\lambda_{max}$ (lg ε) = 279 nm (4.374).
Fluoreszenz (DMSO): $\lambda_{max}$ = 428 nm
$^1$H-NMR ([D]TFA): δ = 8.15 (s, 4H, Phn), 9.81 (s, 4H, Pym-4-H, Pym-6-H), 9.84 (s, 2H, Pym-2-H).

| | | | | |
|---|---|---|---|---|
| $C_{14}H_{10}N_4$ (234.3) | Ber. | C 71.78 | H 4.30 | N 23.92 |
| | Gef. | C 71.72 | H 4.33 | N 23.79 |

Beispiel 7

5,5''-Bis-(phenyl)-2'2'''-(p-phenylen)-d1-(2,5'-bipyrimidin) (20)

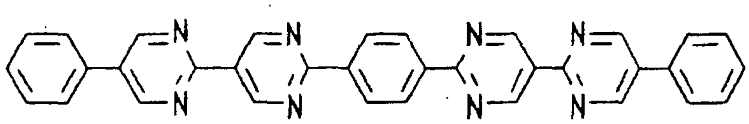

(20)

Die Suspension von 0.80 g (2.10 mmol) Vinamidiniumsalz (14) und 0.63 g (1.05 mmol, 0.5 Äquiv.) Phenylen-1,4-bis(pertrimethylsilylcarbamidin) (R. T. Boere, R. T. Oakley, R. W. Reed, J. Organomet. Chem 331 (1987) 161.) wurde in einem Gemisch aus 30 ml Pyridin und 10 ml DMF unter Zusatz von 0.37 g (6.30 mmol, 3 Äquiv.) Kaliumfluorid 10 h unter Rückfluß zum Sieden erhitzt. Der bereits während des Erhitzens ausgefallene Niederschlag wurde nach dem Abkühlen abgesaugt, mit viel Wasser und anschließend mit Aceton gewaschen. Ausb. 0.54 g (95 %), farbloses Pulver. Umkristallisation von 0.03 g Rohprodukt gelang in 250 ml DMSO mit praktisch quantitativer Ausbeute. Farbloses Pulver, Schmp. > 330°C.
UV/VIS (DMSO): $\lambda_{max}$ (lg ε) = 346 nm, 396 nm (qual.).
Fluoreszenz (DMSO): $\lambda_{max}$ = 489 nm

$^1$H-NMR ([D]TFA): δ = 7.67 - 7.72 (m, 6H, Ph-3-H, Ph-4-H, Ph-5-H), 7.81 - 7.86 (m, 4H, Ph-2-H, Ph-6-H), 8.85 (s, 4H, Phn), 9.62 (s, 4H, Pym), 10.36 (s, 4H, Pym').

| | | | | |
|---|---|---|---|---|
| $C_{34}H_{22}N_8$ (542.6) | Ber. | C 75.26 | H 4.09 | N 20.65 |
| | Gef. | C 75.19 | H 4.07 | N 20.55 |

Beispiel 8

2-(p-Biphenyl)-3-dimethylamino-N,N-dimethyl-prop-2-eniminium-perchlorat (21)

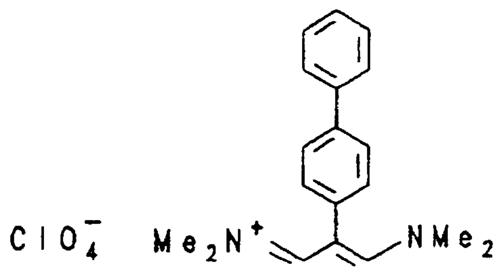

(21)

Zu 54.73 ml (706.71 mmol, 10 Äquiv.) wasserfreiem DMF wurden bei 0°C 16.47 ml (176.68 mmol, 2,5 Äquiv.) Phosphoroxychlorid getropft und das Reaktionsgemisch 30 min bei 0°C gerührt. Anschließend wurden 15.00 g (70.67 mmol) 4-Biphenylessigsäure zugegeben und die Mischung 18 h auf 110°C erwärmt. Nach dem Abkühlen wurde das Reaktionsgemisch mit 70 ml Wasser versetzt, bei Raumtemperatur eine Lösung von 29.78 g (212.01 mmol, 3 Äquiv.) Natriumperchlorat-Monohydrat in 300 ml Wasser zugegeben und der dabei entstandene Niederschlag abgesaugt und mit viel Wasser gewaschen. Ausb. 21.13 g (78 %) beiges Pulver.
$^1$H-NMR ([D]TFA): δ = 2.65 (s, 6H, 2 $NCH_3$), 3.34 (s, 6H, 2 $NCH_3$), 7.34 - 7.49 (m, 7H, 1-H, 3-H und Ph), AA'BB'-Signal zentriert bei 7.67 ($^3J$ = 8 Hz, 2H, PhCCH) und 7.77 ($^3J$ = 8 Hz, 2H, PhCCHCH).

| | | | | |
|---|---|---|---|---|
| $C_{19}H_{23}ClN_2O_4 1/4H_2O$ (383.4) | Ber. | C 59.53 | H 6.18 | N 7.31 |
| | Gef. | C 59.41 | H 5.99 | N 7.09 |

Beispiel 9

5,5'-Bis-(p-biphenyl)-2,2'-(p-phenylen)-di-pyrimidin (23)

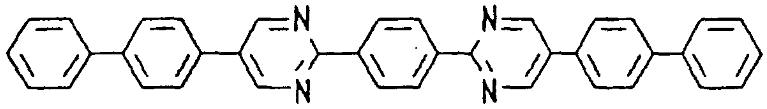

(23)

Die Suspension von 2.00 g (5.22 mmol) Vinamidiniumsalz (21) und 1.55 g (2.61 mmol, 0.5 Äquiv.) Phenylen-1,4-bis(pertrimethylsilylcarbamidin) (R. T. Boere, R. T. Oakley, R. W. Reed, J. Organomet. Chem. 331 (1987) 161) wurde in einem Gemisch aus 45 ml Pyridin und 15 ml DMF unter Zusatz von 0.91 g (15.65 mmol, 3 Äquiv.) Kaliumfluorid 12 h unter Rückfluß zum Sieden erhitzt. Der während des Erhitzens bereits ausgefallene Niederschlag wurde nach dem Abkühlen abgesaugt, mit viel Wasser und anschließend mit Aceton gewaschen. Ausb. 1.34 g (94 %), schwach

gelbes Pulver. Umkristallisation von 0.03 g Rohprodukt gelang in 200 ml DMSO mit nahezu quantitativer Ausbeute. Farbloses Pulver, Schmp. > 330°C.
UV/VIS (DMSO): $\lambda_{max}$ (lg ε) = 347 nm, (qual.).
Fluoreszenz (DMSO): $\lambda_{max}$ = 424 nm

| | | | | |
|---|---|---|---|---|
| $C_{38}H_{26}N_4 \cdot 1/2H_2O$ (547.7) | Ber. | C 83.34 | H 4.97 | N 10.23 |
| | Gef. | C 83.47 | H 4.94 | N 10.19 |

Beispiel 10

2-[2'-(5'-{p-Biphenyl})-pyrimidinyl]-3-dimethylamino-N,N-dimethyl-prop-2-eniminium-perchlorat (24)

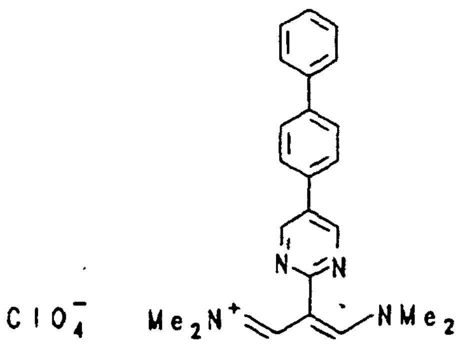

(24)

Zu 11.00 ml (142.10 mmol, 70 Äquiv.) wasserfreiem DMF wurden bei - 50°C unter heftigem Rühren 0.63 ml (6.09 mmol, 3 Äquiv.) Oxalylchlorid getropft. Nach 20 min. Auftauen wurden zu dem Reaktionsgemisch 0.50 g (2.03 mmol) Methylpyrimidin (22) gegeben; die Suspension wurde 28 h auf 50°C erwärmt. Das Reaktionsgemisch wurde nach dem Abkühlen mit 15 ml Wasser versetzt, anschließend die erhaltene klare Lösung bei Raumtemperatur langsam zu 2.85 g (20.30 mmol, 10 Äquiv.) Natriumperchlorat-Monohydrat, gelöst in 200 ml Wasser, getropft. Der dabei entstandene Niederschlag wurde abgesaugt und mit viel Wasser und Methanol gewaschen. Ausb. 0.80 g (86 %) beiges mikrokristallines Pulver.
UV/VIS (DMSO): $\lambda_{max}$ (lg ε) = 321 nm (4.482), sh 380 nm (3.894).
$^1$H-NMR ([D]TFA): δ = 3.45 (s, 6H, 2 $NCH_3$), 3,76 (s, 6H, 2 $NCH_3$), AA'BB'C-Signal zentriert bei 7.41 ($^3J$ = 8 Hz, 1H, Ph-4-H), 7.47 ($^3J$ = 8 Hz, 2H, Ph-3-H, Ph-5-H) und 7.65 ($^3J$ = 8 Hz, 2H, Ph-2-H, Ph-6-H), AA'BB'-Signal zentriert bei 7.77 ($^3J$ = 8 Hz, 2H, PhCCH) und 7.85 ($^3J$ = 8 Hz, 2H, PhCCHC$\underline{H}$), 8.39 (s, 2H, 1-H, 3-H), 9.31 (s, 2H, Pym).

| | | | | |
|---|---|---|---|---|
| $C_{23}H_{25}ClN_4O_4$ (456.9) | Ber. | C 60.46 | H 5.51 | N 12.26 |
| | Gef. | C 61.13 | H 5.53 | N 12.23 |

Beispiel 11

5-(p-Biphenyl)-2'-methyl-2,5'-bipyrimidin (25)

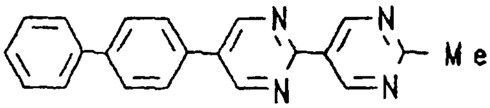

(25)

1.34 g (2.93 mmol) Vinamidiniumsalz (24) wurden mit 0.42 g (4.40 mmol, 1.5 Äquiv.) Acetamidinhydrochlorid in 25 ml Pyridin 12 h unter Rückfluß zum Sieden erhitzt. Nach dem Abkühlen wurde mit 10 ml Methanol versetzt. Der ausgefallene Niederschlag wurde abgesaugt, mit viel Wasser und Methanol gewaschen und aus 50 ml DMSO umkristallisiert. Ausb. 0.79 g (83 %), hellgelbes Pulver, Schmp. 250°C.
UV/VIS (DMSO): $\lambda_{max}$ (lg ε) = 311 nm (4.437), 424 (3.239).
$^1$H-NMR ([D]TFA): δ = 3.26 (s, 3H, $CH_3$), AA'BB'C-Signal zentriert bei 7.42 ($^3J$ = 8 Hz, 1 H, Ph-4-H), 7.49 ($^3J$ = 8 Hz, 2H, Ph-3-H, Ph-5-H) und 7.70 ($^3J$ = 8 Hz, 2H, Ph-2-H, Ph-6-H), AA'BB'-Signal zentriert bei 7.88 ($^3J$ = 8 Hz, 2H, PhCCH) und 7.92 ($^3J$ = 8 Hz, 2H, PhCCHCH),9.57 (s, 2H, Pym), 10.20 ppm (s, 2H, Pym'),

| | | | | |
|---|---|---|---|---|
| $C_{21}H_{16}N_4$ (324.4) | Ber. | C 77.76 | H 4.97 | N 17.27 |
| | Gef. | C 77.64 | H 4.89 | N 17.12 |

Beispiel 12

5,5"-Bis-(p-biphenyl)-2',2'"-(p-phenylen)-di-(2,5'-bipyrimidin) (26)

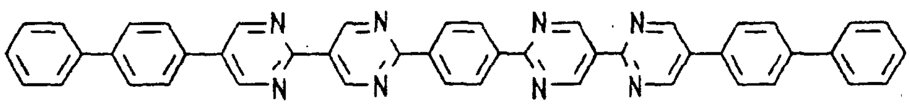

(26)

Die Suspension von 0.70 g (1.53 mmol) Vinamidiniumsalz (24) und 0.46 g (0.77 mmol, 0.5 Äquiv.) Phenylen-1,4-bis(pert-rimethylsilylcarbamidin) (R. T. Boere, R. T. Oakley, R. W. Reed, J. Organomet. Chem 331 (1987) 161.) wurde in einem Lösungsmittelgemisch aus 25 ml Pyridin und 15 ml DMF unter Zusatz von 0.27 g (4.60 mmol, 3 Äquiv.) Kaliumfluorid 15 h unter Rückfluß zum Sieden erhitzt. Der bereits während des Erhitzens ausgefallene, sehr feine Niederschlag wurde nach dem Abkühlen abgesaugt, mit DMF, viel Wasser und Aceton gewaschen. Ausb. = 0.50 g (94 %), gelbes Pulver, Schmp. > 330°C.
MS (70 eV), m/z (%): 694 (100) [$M^+$].

Beispiel 13

5-(p-Biphenyl)-2'-(2''-dimethylamino-ethenyl)-2,5'-bi-pyrimidin (27)

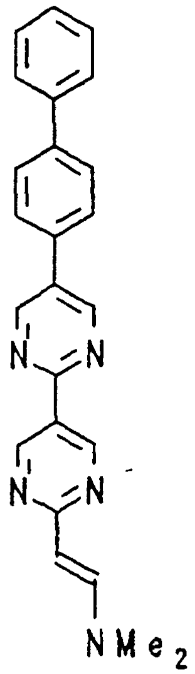

(27)

0.34 g (1.05 mmol) Methylbipyrimidin (25) wurden mit 2.14 ml (10.48 mmol, 10 Äquiv.) tert.-Butyloxy-bis-dimethylaminomethan (Bredereck-Reagenz) 24 h unter schwachem Rühren auf 150°C erhitzt. Nach dem Abkühlen wurde mit 10 ml Isopropanol versetzt. Der ausgefallene Niederschlag wurde abgesaugt und mit Isopropanol und wenig Dichlormethan gewaschen. Ausb. 0.33 g (82 %) gelbes Kristallpulver, Schmelzpunkt 284°C.
UVNIS (DMSO): $\lambda_{max}$ (lg ε) = 297 nm (4.263), 407 (4.780). - $^1$H-NMR ([D]TFA): δ = 3.33 (s, 3H, $CH_3$), 3.56 (s, 3H, $CH_3$), AA'BB'C-Signal zentriert bei 7.44 ($^3J$ = 8 Hz, 1H, Ph-4-H), 7.51 ($^3J$ = 8 Hz, 2H, Ph-3-H, Ph-5-H) und 7.70 ($^3J$ = 8 Hz, 2H, Ph-2-H, Ph-6-H), AA'BB'-Signal zentriert bei 7.86 ($^3J$ = 8 Hz, 2H, PhCCH) und 7.94 ($^3J$ = 8 Hz, 2H, PhCCHCH), 8.73 (s, 1H, $(H_3C)_2$-NCH), AB-Signal zentriert bei 9.30 ($^4J$ = 3 Hz, 1 H, Pym') und 9.53 ($^4J$ = 3 Hz, 1 H, Pym') - protoniertes Pyrimidin (vgl. Vinamidiniumsalz), 9.52 (s, 2H, Pym).

| | | | | |
|---|---|---|---|---|
| $C_{24}H_{21}N_5$ (379.5) | Ber. | C 75.97 | H 5.58 | N 18.46 |
| | Gef. | C 75.86 | H 5.59 | N 18.52 |

Beispiel 14

2-[2''-(5'-p-Biphenyl)-2',5''-bipyrimidinyl]-3-dimethylamino-N,N-dimethyl-prop-2-eniminium-perchlorat (28)

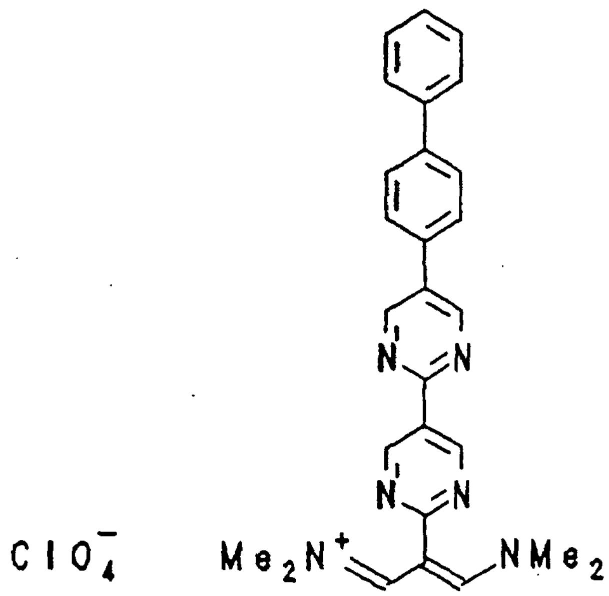

(28)

Zu 4.54 ml (59.03 mmol, 70 Äquiv.) wasserfreiem DMF wurden bei -50°C unter heftigem Rühren 0.11 ml (1.26 mmol, 1.5 Äquiv.) Oxalylchlorid getropft. Zu diesem Reaktionsgemisch wurden anschließend 0.32 g (0.84 mmol) Enamin (27) gegeben; die erhaltene Suspension wurde 6 h bei -50°C gerührt. Nach langsamem Auftauen wurde mit 10 ml Wasser versetzt und die erhaltene klare Lösung bei Raumtemperatur langsam zu 1.18 g (8.43 mmol, 10 Äquiv.) Natriumperchlorat-Monohydrat, gelöst in 100 ml Wasser, getropft. Der dabei entstandene Niederschlag wurde abgesaugt und mit viel Wasser und Methanol gewaschen. Ausb. 0.36 g (80 %) beiges mikrokristallines Pulver.
UV/VIS (DMSO): $\lambda_{max}$ (lg $\varepsilon$) = 360 nm (4.505), sh 429 (3.907). - $^1$H-NMR ([D]TFA): $\delta$ = 3.54 (s, 6H, 2 $NCH_3$), 3,85 (s, 6H, 2 $NCH_3$), AA'BB'C-Signal zentriert bei 7.42 ($^3$J = 8 Hz, 1H, Ph-4-H), 7.49 ($^3$J = 8 Hz, 2H, Ph-3-H, Ph-5-H) und 7.70 ($^3$J = 8 Hz, 2H, Ph-2-H, Ph-6-H), AA'BB'-Signal zentriert bei 7.88 ($^3$J = 8 Hz, 2H, PhCCH) und 7.93 ($^3$J = 8 Hz, 2H, PhCCHCH), 8.65 (s, 2H, 1-H, 3-H), 9.61 (s, 2H, Pym'), 9.96 (s, 2H, Pym'').

Beispiel 15

5,5'''-Bis(p-biphenyl)-2'',2'''''-(p-biphenylen)di-(2,5';-2',5''-terpyrimidin) (29)

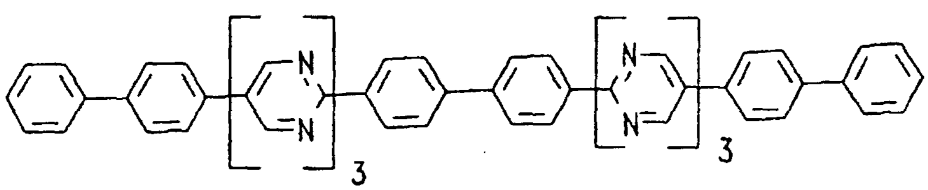

(29)

Eine Suspension von 0.34 g (0.64 mmol) Vinamidiniumsalz (28) und 0.21 g (0.32 mmol, 0.5 Äquiv.) 1,1'-Biphenylen-4,4'-bis(pertrimethylsilylcarbamidin) wurde in einem Lösungsmittelgemisch aus 20 ml Pyridin und 15 ml DMF unter Zusatz von 0.11 g (1.91 mmol, 3 Äquiv.) Kaliumfluorid 15 h unter Rückfluß zum Sieden erhitzt. Der bereits

während des Erhitzens ausgefallene, sehr feine Niederschlag wurde nach dem Abkühlen abgesaugt und mit DMF, viel Wasser und Aceton gewaschen. Anschließend wurde das Produkt mit DMSO ausgekocht. Ausb. = 0.27 g (92 %), braungelbes Pulver,
Schmp. > 330°C.
UV/VIS (TFA): $\lambda_{max}$ (lg ε) = 386 nm (4.892). - MS (70 eV), m/z (%): 926 (55) [M'];

**Patentansprüche**

**1.** Verwendung von konjugierten Verbindungen, welche zwei oder mehr Pyrimidinringe als Teil des konjugierten Systems enthalten, gemäß der Formel (I)

$$R^1\text{-}F^1_a\text{-}D^1_b\text{-}B^1_c\text{-}A_d\text{-}C^1_e\text{-}E_f\text{-}G_g\text{-}R^2 \qquad \text{(I)}$$

wobei die Symbole und Indizes folgende Bedeutungen haben,

A ist

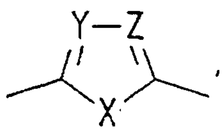

Biphenyl-4,4'-diyl, Anthracen-9,10-diyl, Pyrimidin-2,5-diyl, 1,4-Phenylen;

X ist -O-, -CH = CH-, -CH = N-, -N = CH-;

Y, Z sind gleich oder verschieden $-CR^3$ =, -N =;

$B^1$, $C^1$ sind gleich oder verschieden Pyrimidin-2,5-diyl, 1,4-Phenylen, wobei ein oder zwei Wasserstoffatome durch Reste $R^3$ substituiert sein können, Pyridin-2,5-diyl, Pyridinium-1,4-diyl, Pyridinium-2,5-diyl, wobei der Stickstoff H, eine Alkylgruppe mit 1 bis 20 Kohlenstoffatomen oder eine unsubstituierte Phenylgruppe tragen kann, $-CR^4 = CR^5-$, -C ≡ C-;

$D^1$, E sind gleich oder verschieden A, $B^1$, $C^1$, $-B^1\text{-}A\text{-}C^1-$,

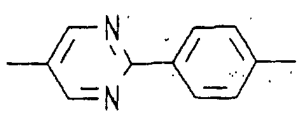 , 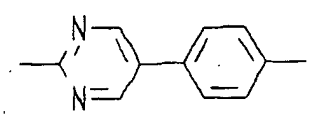 , 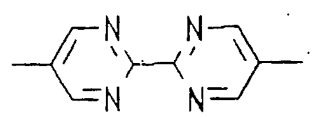 ,

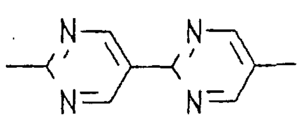 , 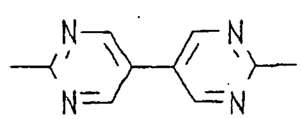 ;

$F^1$, G sind gleich oder verschieden $D^1$, E, Pyridin-2,5-diyl, Pyridinium-1,4-diyl, Pyridinium-2,5-diyl, wobei der Stickstoff H, eine Alkylgruppe mit 1 bis 20 Kohlenstoffatomen oder eine unsubstituierte Phenylgruppe tragen kann, 4-Pyridyl;

$R^1$,$R^2$,$R^3$,$R^4$,$R^5$ sind gleich oder verschieden H, eine unverzweigte oder verzweigte Alkylgruppe mit 1 bis 12 Kohlenstoffatomen, wobei eine oder mehrere $-CH_2-$Gruppen durch -O- ersetzt sein können,

-CN, $-NR_2^6$;

| | |
|---|---|
| $R^6$ | ist H, eine Alkylgruppe mit 1 bis 6 Kohlenstoffatomen, Phenyl; |
| a, c, d, e, g | sind gleich oder verschieden 0 oder 1; |
| b, f | sind gleich oder verschieden 0, 1 oder 2; |

wobei die Summe a+b+c+d+e+f+g mindestens 3 sein muß und die Gruppe $-F_a^1-D_b^1-B_c^1-A_d-C_e^1-E_f-G_g-$ mindestens 2 Pyrimidin-2,5-diyl-Gruppen enthalten muß, als Elektrolumineszenzmaterialien.

**2.** Verwendung nach Anspruch 1, **dadurch gekennzeichnet, daß** die Symbole und Indizes in der Formel (I) folgende Bedeutungen haben:

| | |
|---|---|
| A | ist Pyrimidinyl-2,5-diyl, 1,4-Phenylen; |
| $B^1$, $C^1$ | sind gleich oder verschieden Pyrimidin-2,5-diyl, 1,4-Phenylen, wobei ein oder zwei Wasserstoffatomen durch Reste $R^3$ substituiert sein können, -CH = CH-; |
| $D^1$, E | sind gleich oder verschieden A, $B^1$, $C^1$, $-B^1-A-C^1-$, |

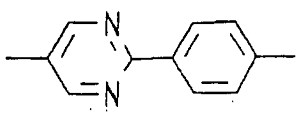 , 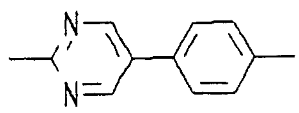 , 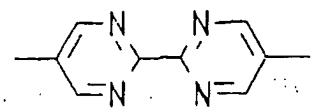 ,

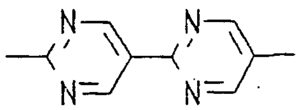 , 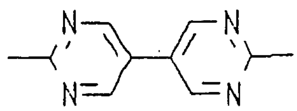 ;

| | |
|---|---|
| $F^1$, G | sind gleich oder verschieden $D^1$, E; |
| $R^1$,$R^2$,$R^3$ | sind gleich oder verschieden H, eine unverzweigte oder verzweigte Alkylgruppe mit 1 bis 12 Kohlenstoffatomen, wobei eine $-CH_2$-Gruppe durch -O- ersetzt sein kann, -CN, $-NR_2^6$; |
| $R^6$ | ist H, eine Alkylgruppe mit 1 bis 6 Kohlenstoffatomen, Phenyl; |
| a, c, d, e, g | sind gleich oder verschieden 0 oder 1; |
| b, f | sind gleich oder verschieden 0, 1 oder 2; |

wobei die Summe a + b + c + d + e + f + g mindestens 3 sein muß und die Gruppe $-F_a^1-D_b^1-B_c^1-A_d-C_e^1-E_f-G_g-$ mindestens 2 Pyrimidin-2,5-diyl-Gruppen enthalten muß.

**3.** Verwendung nach einem oder mehreren der Ansprüche 1, **dadurch gekennzeichnet, daß** die Symbole und Indizes in der Formel (I) folgende Bedeutungen haben:

| | |
|---|---|
| A | ist Pyrimidinyl-2,5-diyl, 1,4-Phenylen; |
| $B^1$, $C^1$ | sind gleich oder verschieden Pyrimidin-2,5-diyl, 1 ,4-Phenylen, -CH≡CH-; |
| $D^1$, E | sind gleich oder verschieden A, $B^1$, $C^1$, $-B^1A-C^1-$, |

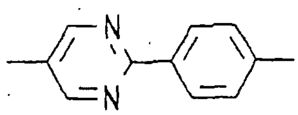 , 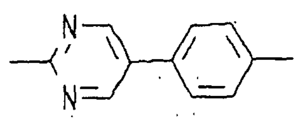 , 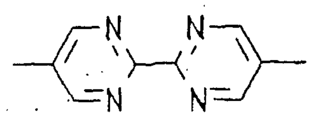 ,

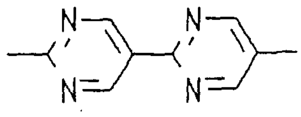 , 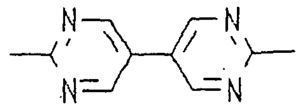 ;

| | |
|---|---|
| $F^1$, G | sind gleich oder verschieden $D^1$, E; |
| $R^1$,$R^2$ | sind H, eine unverzweigte oder verzweigte Alkylgruppe mit 1 bis 12 Kohlenstoffatomen, $-NR_2^6$; |
| $R^6$ | ist H, eine Alkylgruppe mit 1 bis 6 Kohlenstoffatomen, Phenyl; |
| a, c, d, e, g | sind gleich oder verschieden 0 oder 1; |
| b, f | sind gleich oder verschieden 0, 1 oder 2; |

wobei die Summe a + b + c + d + e + f + g mindestens 3 sein muß und die Gruppe $-F^1_a-D^1_b-B^1_c-A_d-C^1_e-E_f-G_g-$ mindestens 2 Pyrimidin-2,5-diyl-Gruppen enthalten muß.

**4.** Verwendung nach einem oder mehreren der Ansprüche 1 bis 3, **dadurch gekennzeichnet, daß** die Gruppe $-F^1_a-D^1_b-B^1_c-A_d-C^1_e-E_f-G_g-$ in der Formel (I) ausgewählt ist aus der Gruppe:

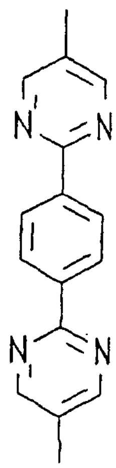

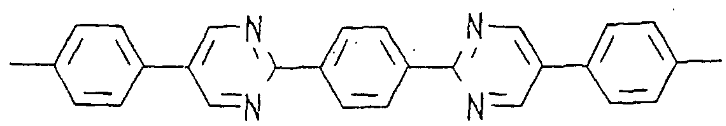

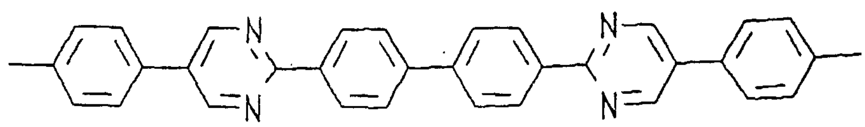

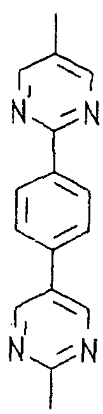

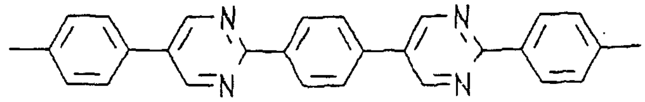

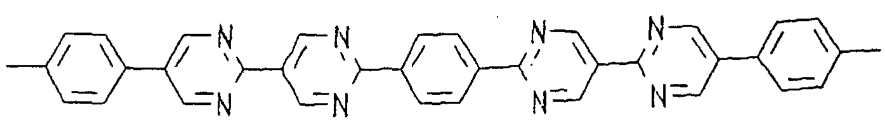

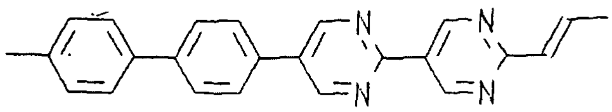,

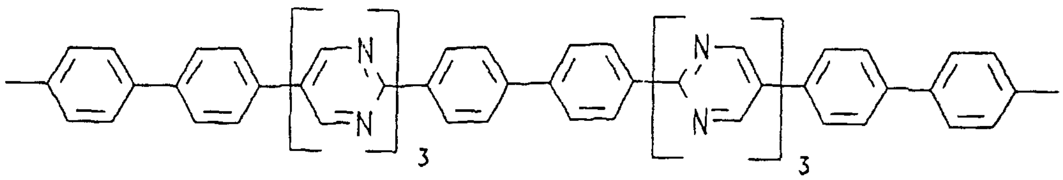,

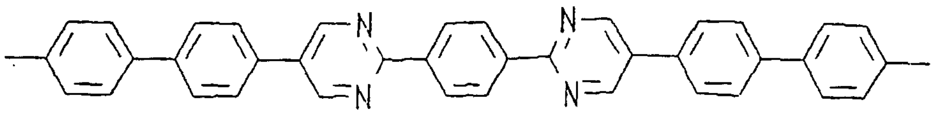

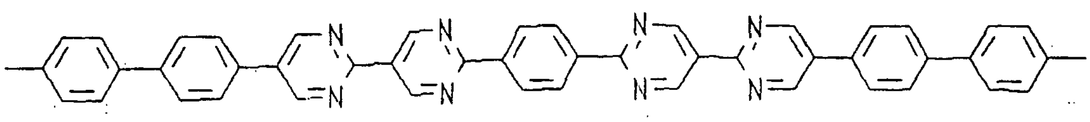

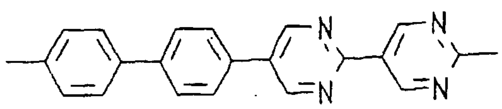

und $R^1$ und $R^2$ die in der Formel (I) angegebenen Bedeutungen haben.

**5.** Elektrolumineszenzmaterial, bestehend aus einer Verbindung nach einem oder mehreren der Ansprüche 1 bis 4.

**6.** Elektrolumineszenzvorrichtung, enthaltend als eine aktive Schicht ein Elektrolumineszenzmaterial, bestehend aus einer Verbindung nach einem oder mehreren der Ansprüche 1 bis 4.

**7.** Pyrimidinverbindung der Formel (I),

$$R^1\text{-}F^1_a\text{-}D^1_b\text{-}B^1_c\text{-}A_d\text{-}C^1_e\text{-}E_f\text{-}G_g\text{-}R^2 \qquad (I)$$

worin die Symbole und Indizes folgende Bedeutungen haben:

A ist

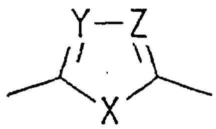 ,

Biphenyl-4,4'-diyl, Anthracen-9,10-diyl, Pyrimidin-2,5-diyl, 1,4-Phenylen;

X ist -O-, -CH = CH-, -CH = N-, -N = CH-;

Y, Z sind gleich oder verschieden $-CR^3 =$, -N =;

$B^1$, $C^1$ sind gleich oder verschieden Pyrimidin-2,5-diyl, 1,4-Phenylen, wobei ein oder zwei Wasserstoffatome durch Reste $R^3$ substituiert sein können, Pyridin-2,5-diyl, Pyridinium-1,4-diyl, Pyridinium-2,5-diyl, wobei der Stickstoff H, eine Alkylgruppe mit 1 bis 20 Kohlenstoffatomen oder eine unsubstituierte oder substituierte Phenylgruppe tragen kann, $-CR^4 = CR^5-$, -C≡C-;

$D^1$, E sind gleich oder verschieden A, $B^1$, $C^1$, $-B^1-A-C^1-$;

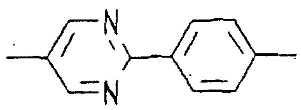 , 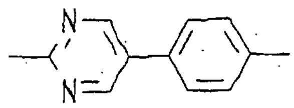 , 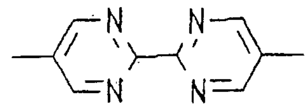 ,

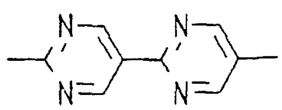 , 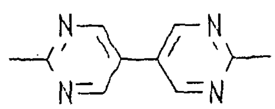 ;

$F^1$, G sind gleich oder verschieden $D^1$, E, Pyridin-2,5-diyl, Pyridinium-1,4-diyl, Pyridinium-2,5-diyl, wobei der Stickstoff H, eine Alkylgruppe mit 1 bis 20 Kohlenstoffatomen oder eine unsubstituierte oder substituierte Phenylgruppe tragen kann, 4-Pyridyl;

$R^1$,$R^2$,$R^3$,$R^4$,$R^5$ sind gleich oder verschieden H, eine unverzweigte oder verzweigte Alkylgruppe mit 1 bis 12 Kohlenstoffatomen, wobei eine oder mehrere $-CH_2$-Gruppen durch -O- ersetzt sein können, -CN, $-NR_2^6$;

$R^6$ ist H, eine Alkylgruppe mit 1 bis 6 Kohlenstoffatomen, Phenyl;

a, c, d, e, g sind gleich oder verschieden 0 oder 1;

b, f sind gleich oder verschieden 0, 1 oder 2,

mit den Maßgaben, daß a) die Summe a+b+c+d+e+f+g mindestens 5 ist und/oder daß b) die Summe a + b + c + d + e + f + g mindestens 3 ist und daß die Gruppe $-F^1_a-D^1_b-B^1_c-A_d-C^1_e-E_f-G_g-$ mindestens 3 Pyrimidin-2,5-diyl-Gruppen enthält.

**8.** Pyrimidinverbindung nach Anspruch 7, **dadurch gekennzeichnet, daß** bei den Verbindungen der Formel (I) die Symbole und Indizes folgende Bedeutungen haben:

A ist Pyrimidin-2,5-diyl, 1,4-Phenylen;

$B^1$, $C^1$ sind gleich oder verschieden Pyrimidin-2,5-diyl, 1,4-Phenylen, wobei ein oder zwei Wasserstoffatome durch Reste $R^3$ substituiert sein können, -CH = CH-;

$D^1$, E sind gleich oder verschieden A, $B^1$, $C^1$, $-B^1-A-C^1-$,

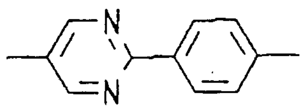 , 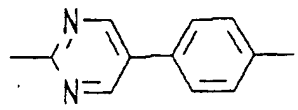 , 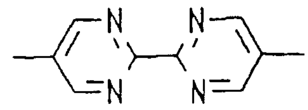 ,

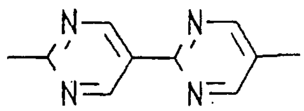 , 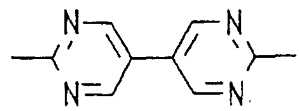 ;

| | |
|---|---|
| $F^1$, G | sind gleich oder verschieden $D^1$, E; |
| $R^1$,$R^2$,$R^3$ | sind gleich oder verschieden H, eine unverzweigte oder verzweigte Alkylgruppe mit 1 bis 12 Kohlenstoffatomen, wobei eine $-CH_2$-Gruppe durch -O- ersetzt sein kann, -CN, $-NR_2^6$; |
| $R^6$ | ist H, eine Alkylgruppe mit 1 bis 6 Kohlenstoffatomen, Phenyl; |
| a, c, d, e, g | sind gleich oder verschieden 0 oder 1; |
| b, f | sind gleich oder verschieden 0, 1 oder 2; |

mit den Maßgaben, daß a) die Summe a + b + c + d + e + f + g 5 bis 16 ist und/oder daß b) die Summe a + b + c + d + e + f + g mindestens 3 ist und daß die Gruppe $-F^1_a-D^1_b-B^1_c-A_d-C^1_e-E_f-G_g-$ mindestens 3 Pyrimidin-2,5-diyl-Gruppen enthält.

## Claims

**1.** Use of conjugated compounds which contain two or more pyrimidine rings as part of the conjugated system and in accordance with Formula (1):

$$R^1 - F^1{}_a - D^1{}_b - B^1{}_c - A_d - C^1{}_e - E_f - G_g - R^2 \qquad (I)$$

whereby the symbols and indices have the following significances

A is

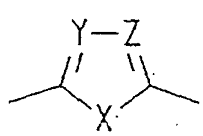

biphenyl-4-4'-diyl, anthracene -9,10-diyl, pyrimidine-2,5-diyl, 1,4-phenylene;

| | |
|---|---|
| X | is -O-, -CH=CH-, -CH-=N-, -N=CH-; |
| Y, Z | are the same or different- $-CR^3=$, -N= ; |
| $B^1$ , $C^1$ | are the same or different - pyrimidine-2,5-diyl, 1,4-phenylene, whereby one or two hydrogen atoms can be substituted by $R^3$ residues, pyridine-2,5-diyl, pyridinium-1,4-diyl, pyridinium-2,5-diyl, whereby the nitrogen can carry hydrogen, an alkyl group with 1 to 20 carbon atoms or an unsubstituted phenyl group, $-CR^4=CR^5-$, -C≡C-; |
| $D^1$, E | are the same or different - A, $B^1$, $C^1$, $B^1$-A-$C^1$ , |

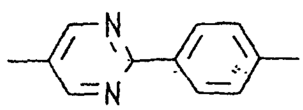 , 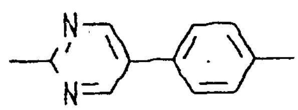 , 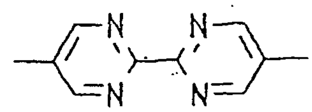 ,

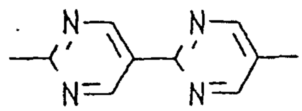 , 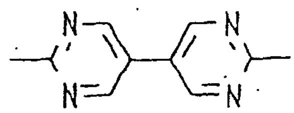 ;

$F^1$, G — are the same or different - $D^1$, E, pyridine-2,5-diyl, pyridinium-1,4-diyl, pyridinium-2,5-diyl, whereby the nitrogen can carry hydrogen, an alkyl group with 1 to 20 carbon atoms or an unsubstituted phenyl group, 4-pyridyl;

$R^1$, $R^2$, $R^3$, $R^4$, $R^5$ — are the same or different - hydrogen, an unbranched or branched alkyl Group with 1 to 12 carbon atoms, whereby one or several $-CH_2$ groups can be replaced by - O-, -CN, $-NR_2^6$;

$R^6$ — is H, an alkyl group with 1 to 6 carbon atoms, phenyl;

a, c, d, e, g — are the same or different - 0 or 1;

b, f — are the same or different - 0, 1 or 2;

whereby the sum of a + b + c + d + e + f + g must equal a minimum of 3 and the group $- F^1_a - D^1_b - B^1_c - A_d - C^1_e - E_f - G_g -$ must contain a minimum of 2 pyrimidine-2,5-diyl-groups as electro-luminescent material.

**2.** Use in accordance with claim 1, **characterised in that** the symbols and indices in Formula (1) have the following significances:

A — is pyrimidinyl-2,5-diyl, 1,4-phenylene;

$B^1$, $C^1$ — are the same or different - pyrimidine-2,5-diyl, 1,4-phenylene, whereby one or two hydrogen atoms can be substituted by $R^3$-residues, -CH=CH- ;

$D^1$, E — are the same or different - A, $B^1$, $C^1$, $-B^1-A-C^1-$;

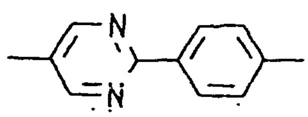 , 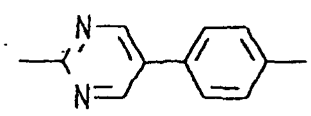 , 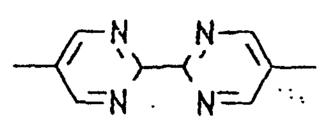 ,

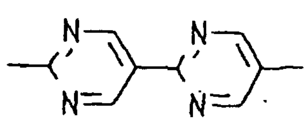 , 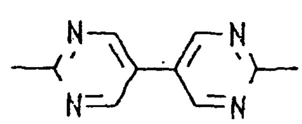 ;

$F^1$, G — are the same or different - $D^1$, E;

$R^1$, $R^2$, $R^3$, — are the same or different - hydrogen, an unbranched or branched alkyl group with 1 to 12 carbon atoms, whereby one $-CH_2$ group can be replaced by -O-, - CN, $-NR_2^6$ ;

$R^6$ — is hydrogen, an alkyl group with 1 to 6 carbon atoms, phenyl ;

a, c, d, e, g — are the same or different - 0 or 1;

b, f — are the same or different - 0, 1 or 2;

whereby the sum of a + b + c + d + e + f + g must equal a minimum of 3 and the group $- F^1_a - D^1_b - B^1_c - A_d - C^1_e - E_1 - G_g -$ must contain a minimum of 2 pyrimidine-2,5-diyl-groups.

**3.** Use according to one or more of claims 1, **characterised in that** the symbols and indices in formula (I) have the following significances:

| | |
|---|---|
| A | is pyrimidinyl-2,5-diyl, 1,4-phenylene; |
| $B^1$, $C^1$ | are the same or different - pyrimidine-2,5-diyl, 1,4-phenylene, -CH≡CH- ; |
| $D^1$, E | are the same or different - A, $B^1$, $C^1$, $-B^1$-A-$C^{1-}$; |

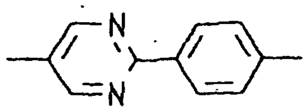 , 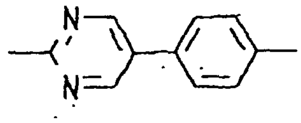 , 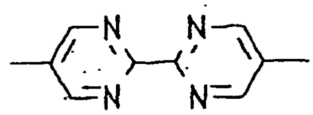 ,

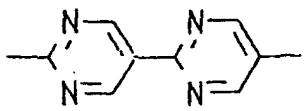 , 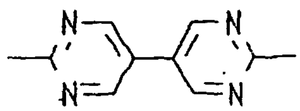 ;

| | |
|---|---|
| $F^1$, G | are the same or different - $D^1$, E |
| $R^1$, $R^2$ | are H, an unbranched or branched alkyl group with 1 to 12 carbon atoms, $-NR_2^6$; |
| $R^6$ | is H, an alkyl group with 1 to 6 carbon atoms, phenyl; |
| a, c, d, e, g | are the same or different - 0 or 1; |
| b, f | are the same of different - 0, 1 or 2; |

whereby the sum of a + b + c + d + e + f + g must equal a minimum of 3 and the group $- F^1_a - D^1_b - B^1_c - A_d - C^1_e - E_1 - G_g -$ must contain a minimum of 2 pyrimidine-2,5-diyl-groups.

**4.** Use of one or more of the claims 1 to 3, **characterised in that** the group $F^1_a - D^1_b - B^1_c - A_d - C^1_e - E_1 - G_g$ in Formula (I) is selected from the group

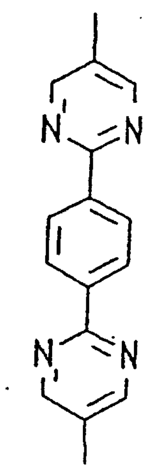

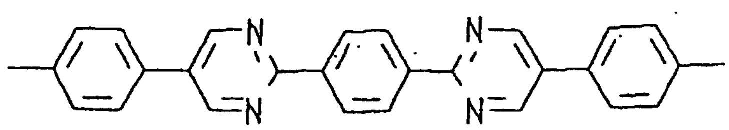

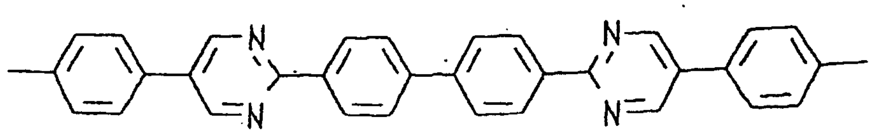

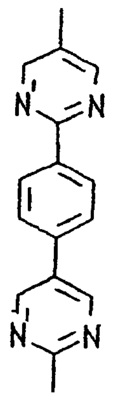

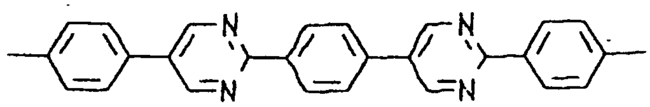

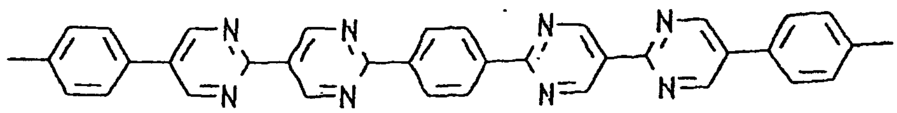

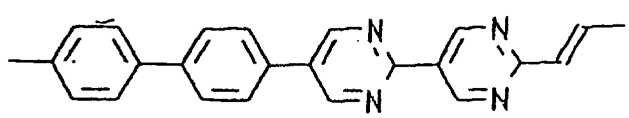

,

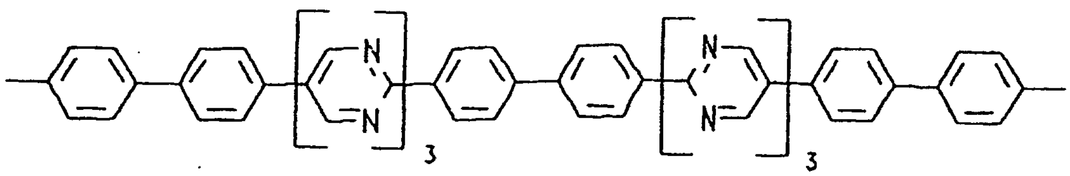

,

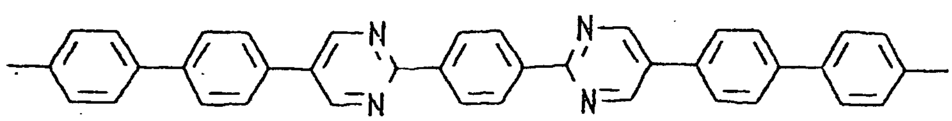

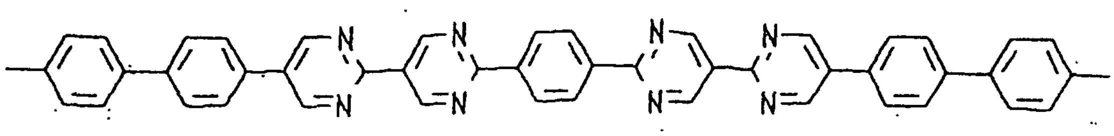

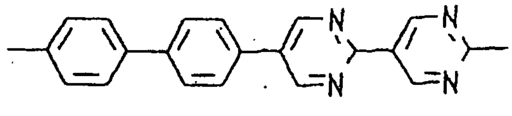

and $R^1$ and $R^2$ have the significances given in Formula (I).

**5.** Electro-luminescent material consisting of a compound according to one or more of the claims 1 to 4.

**6.** An electro-luminescent device containing an electro-luminescent material as an active layer, consisting of a compound according to one or more of the claims 1 to 4.

**7.** A pyrimidine compound of the Formula (I)

$$R^1 - F^1{}_a - D^1{}_b - B^1{}_c - A_d - C^1{}_e - E_f - G_g - R^2 \qquad (I)$$

whereby the symbols and indices have the following significances :

A

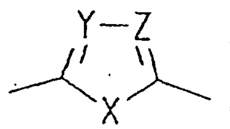

is biphenyl-4-4'-diyl, anthracene-9, 10-diyl, pyrimidine-2,5-diyl, 1,4-phenylene;

X is -O-, -CH=CH-, -CH=N, -N=CH-;

Y, Z are the same or different - $-CR^3=$, -N=;

$B^1$, $C^1$ are the same or different - pyrimidine-2,5-diyl, 1,4-phenylene, where by one or two hydrogen atoms can be substituted by $R^3$ residues, pyridine-2,5-diyl, pyridinium-1,4-diyl, pyridinium-2,5-diyl, whereby the nitrogen can carry H, an alkyl group with 1 to 20 C-atoms or an unsubstituted or substituted phenyl group, $-CR^4=CR^5$, -C≡C-;

$D^1$, E are the same or different - A, $B^1$, $C^1$, $-B^1$-A-$C^1$-;

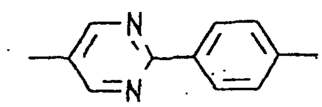 , 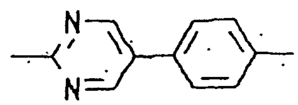 , 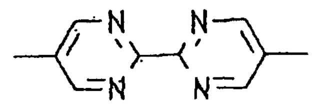 ,

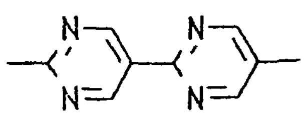 , 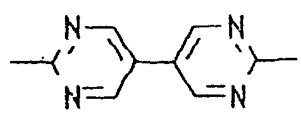 ;

$F^1$, G are the same or different- $D^1$, E, pyridine-2,5-diyl, pyridinium-1,4-diyl, pyridinium-2,5-diyl, whereby the nitrogen can carry H, an alkyl group with 1 to 20 carbon atoms or an unsubstituted or substituted phenyl group, 4-pyridyl;

$R^1$, $R^2$, $R^3$, $R^4$, $R^5$ are the same or different - Hydrogen, an unbranched or branched alkyl group with 1 to 12 carbon atoms, whereby one or several $-CH_2$ groups can be replaced by -O-, -CN, $-NR_2{}^6$;

$R^6$ is H, an alkyl group with 1 to 6 carbon atoms, phenyl ;

a, c, d, e, g are the same or different - 0 or 1;

b, f are the same or different - 0, 1 or 2;

with the requirement that a) the sum of a + b + c + d + e + f + g must equal a minimum of 5 and / or that the sum of a + b + c + d + e + f + g must equal a minimum of 3 and that the group $- F^1{}_a - D^1{}_b - B^1{}_c - A_d - C^1{}_e - E_f - G_g -$ must contain a minimum of 3 pyrimidine-2,5-diyl-groups.

**8.** Pyrimidine compound in accordance with claim 7, **characterised in that** in the compounds of Formula (I) the symbols and indices have the following significances:

| | |
|---|---|
| A | is pyrimidine-2,5-diyl, 1,4-phenylene; |
| $B^1$, $C^1$ | are the same or different - pyrimidine-2,5-diyl, 1,4-phenylene, whereby one or two hydrogen atoms may be substituted by $R^3$-residues, -CH=CH= ; |
| $D^1$, E | are the same or different - A, $B^1$, $C^1$, $-B^1-A-C^1-$; |

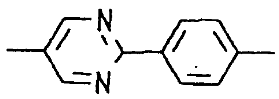 , 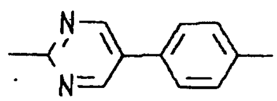 , 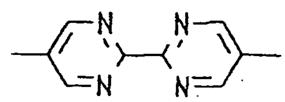 ,

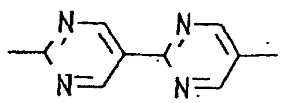 , 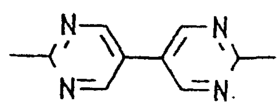 ;

| | |
|---|---|
| $F^1$, G | are the same or different - $D^1$, E; |
| $R^1$, $R^2$, $R^3$ | are the same or different - hydrogen, an unbranched or branched alkyl group with 1 to 12 carbon atoms, whereby one $-CH_2$ group can be replaced by -O-, - CN, $-NR_2^6$. |
| $R^6$ | is H, an alkyl group with 1 to 6 carbon atoms, phenyl; |
| a, c, d, e, g | are the same or different - 0 or 1; |
| b, f | are the same or different - 0, 1 or 2; |

with the requirement that a) the sum of a + b + c + d + e + f + g is 5 to 16 and / or that the sum of a + b + c + d + e + f + g must equal a minimum of 3 and that the group $- F^1_a - D^1_b - B^1_c - A_d - C^1_e - E_f - G_g -$ must contain a minimum of 3 pyrimidine-2,5-diyl-groups.

## Revendications

**1.** Utilisation de composés conjugués, qui contiennent deux noyaux pyrimidine ou plus comme partie du système conjugué,

$$R^1-F^1{}_a-D^1{}_b-B^1{}_c-A_d-C^1{}_e-E_f-G_g-R^2 \qquad (I)$$

les symboles et indices ayant les significations suivantes,

A représente

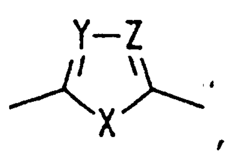

un groupe biphényl-4,4'-diyle, un groupe anthracène-9,10-diyle, un groupe pyrimidine-2,5-diyle, un groupe 1,4-phénylène ;
X représente -O-, -CH=CH-, -CH=N-, -N-=CH- ;
Y, Z, identiques ou différents, représentent $-CR^3=$, -N= ;

$B^1$, $C^1$, identiques ou différents, représentent un groupe pyrimidine-2,5-diyle, un groupe 1,4-phénylène, un ou deux atomes d'hydrogène pouvant être substitués par des restes $R^3$, un groupe pyridine-2,5-diyle, un groupe pyridinium-1,4-diyle, un groupe pyridinium-2,5-diyle, l'azote pouvant porter un atome d'hydrogène, un groupe alkyle de 1 à 20 atomes de carbone ou un groupe phényle non substitué, $-CR^4=CR^5-$, $-C\equiv C-$ ;
$D^1$, E, identiques ou différents, représentent A, $B^1$, $C^1$, $-B^1-A-C^1-$,

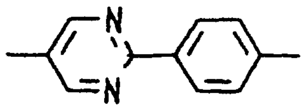 , 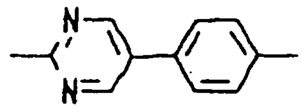 , 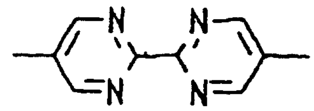 ,

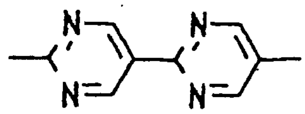 , 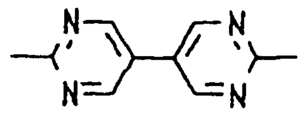 ;

$F^1$, G, identiques ou différents, représentent $D^1$, E, un groupe pyridine-2,5-diyle, un groupe pyridinium-1,4-diyle, un groupe pyridinium-2,5-diyle, l'azote pouvant porter un atome d'hydrogène, un groupe alkyle de 1 à 20 atomes de carbone ou un groupe phényle non substitué, un groupe 4-pyridyle ;
$R^1$, $R^2$, $R^3$, $R^4$, $R^5$, identiques ou différents, représentent H, un groupe alkyle non ramifié ou ramifié de 1 à 12 atomes de carbone, un ou plusieurs groupes $-CH_2-$ pouvant être remplacés par -O-, -CN, $-NR_2^6$ ;
$R^6$ représente H, un groupe alkyle de 1 à 6 atomes de carbone, un groupe phényle ;
a, c, d, e, g, identiques ou différents, valent 0 ou 1 ;
b, f, identiques ou différents, valent 0, 1 ou 2 ;

la somme a+b+c+d+e+f+g devant valoir au moins 3 et le groupe $-F^1_a-D^1_b-B^1_c-A_d-C^1_e-E_f-G_g$ devant contenir au moins 2 groupes pyrimidine-2,5-diyle, comme matériaux électroluminescents.

2. Utilisation selon la revendication 1, **caractérisée en ce que** les symboles et indices dans la formule (I) ont les significations suivantes :

A représente un groupe pyrimidinyle-2,5-diyle, un groupe 1,4-phénylène ;
$B^1$, $C^1$, identiques ou différents, représentent un groupe pyrimidine-2,5-diyle, un groupe 1,4-phénylène, un ou deux atomes d'hydrogène pouvant être substitués par un reste $R^3$, -CH=CH- ;
$D^1$, E identiques ou différents représentent A, $B^1$, $C^1$, $-B^1-A-C^1-$,

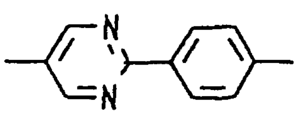 , 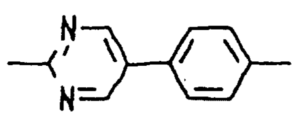 , 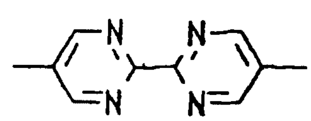 ,

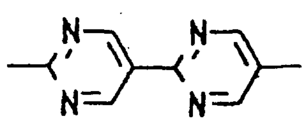 , 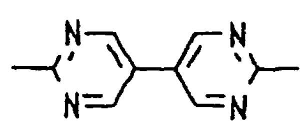 ;

$F^1$, G, identiques ou différents, représentent $D^1$, E ;
$R^1$, $R^2$, $R^3$, identiques ou différents, représentent H, un groupe alkyle non ramifié ou ramifié de 1 à 12 atomes de carbone, un ou plusieurs groupes $-CH_2-$ pouvant être remplacés par -O-, -CN, $-NR_2^6$ ;
$R^6$ représente H, un groupe alkyle de 1 à 6 atomes de carbone, un groupe phényle ;
a, c, d, e, g, identiques ou différents, valent 0 ou 1 ;
b, f, identiques ou différents, valent 0, 1 ou 2 ;

la somme a+b+c+d+e+f+g devant valoir au moins 3 et le groupe $-F^1_a-D^1_b-B^1_c-A_d-C^1_e-E_f-G_g$ devant contenir au moins 2 groupes pyrimidine-2,5-diyle.

**3.** Utilisation selon la revendication 1, **caractérisée en ce que** les symboles et indices de la formule (I) ont les significations suivantes :

A représente un groupe pyrimidinyle-2,5-diyle, un groupe 1,4-phénylène ;
$B^1$, $C^1$, identiques ou différents, représentent un groupe pyrimidine-2,5-diyle, un groupe 1,4-phénylène, -CH≡CH- ;
$D^1$, E, identiques ou différents, représentent A, $B^1$, $C^1$, $-B^1-A-C^1-$ ;

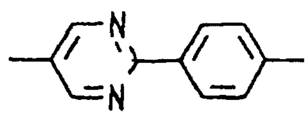 , 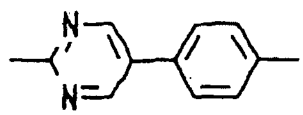 , 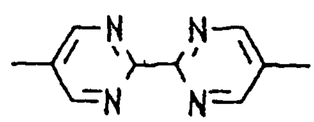 ,

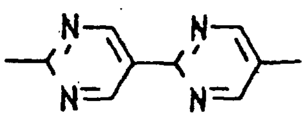 , 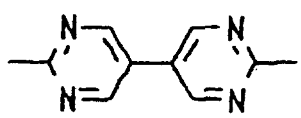 ;

$F^1$, G, identiques ou différents, représentent $D^1$, E ;
$R^1$, $R^2$, identiques ou différents, représentent H, un groupe alkyle non ramifié ou ramifié de 1 à 12 atomes de carbone, $-NR_2^6$ ;
$R^6$ représente H, un groupe alkyle de 1 à 6 atomes de carbone, un groupe phényle ;
a, c, d, e, g, identiques ou différents, valent 0 ou 1 ;
b, f, identiques ou différents, valent 0, 1 ou 2 ;

la somme a+b+c+d+e+f+g devant valoir au moins 3 et le groupe $F^1_a-D^1_b-B^1_c-A_d-C^1_e-E_f-G_g$ devant contenir au moins 2 groupes pyrimidine-2,5-diyle.

**4.** Utilisation selon l'une quelconque des revendications 1 à 3, **caractérisée en ce que** le groupe $-F^1_a-D^1_b-B^1_c-A_d-C^1_e-E_f-G_g$ dans la formule (I) est choisi dans le groupe :

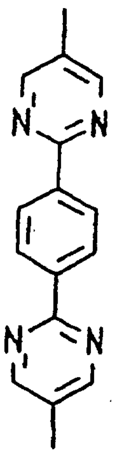

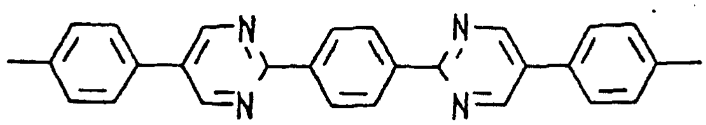

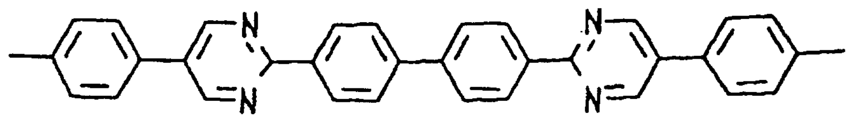

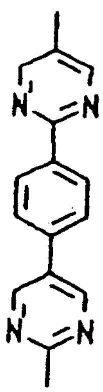

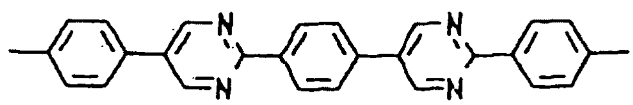

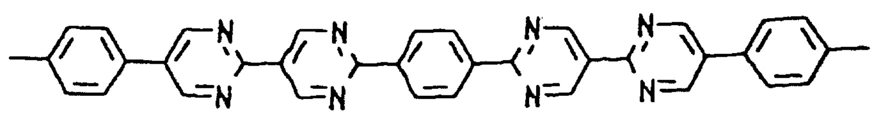

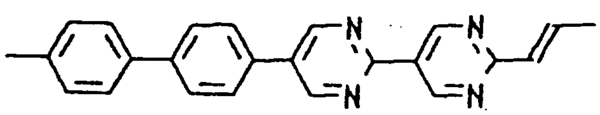

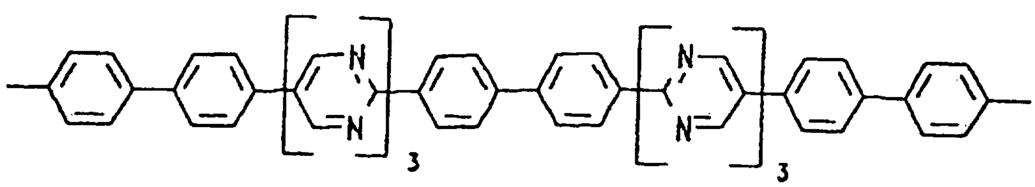

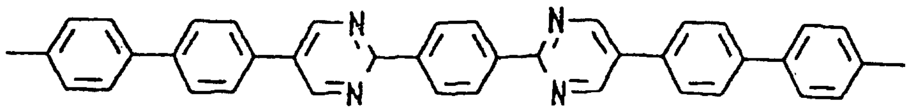

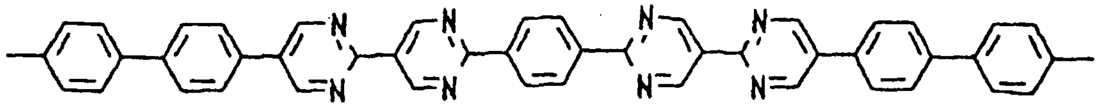

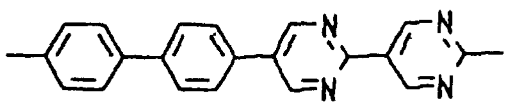

et $R^1$ et $R^2$ qui ont les significations données dans la formule (I).

**5.** Matériau électroluminescent, comportant un composé selon l'une quelconque des revendications 1 à 4.

**6.** Dispositif électroluminescent, comportant comme couche active un matériau électroluminescent à base d'un composé selon l'une quelconque des revendications 1 à 4.

**7.** Composé pyrimidine répondant à la formule (I),

$$R^1\text{-}F^1{}_a\text{-}D^1{}_b\text{-}B^1{}_c\text{-}A_d\text{-}C^1{}_e\text{-}E_f\text{-}G_g\text{-}R^2 \qquad (I)$$

dans laquelle les symboles et indices ont les significations suivantes :

A représente

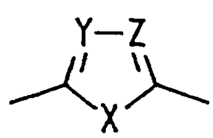

,

un groupe biphényl-4,4'-diyle, un groupe anthracène-9,10-diyle, un groupe pyrimidine-2,5-diyle, 1,4-phénylène ;
X représente -O-, -CH=CH-, -CH=N-, -N-=CH- ;
Y, Z, identiques ou différents, représentent $-CR^3=$, -N= ;
$B^1$, $C^1$ identiques ou différents représentent un groupe pyrimidine-2,5-diyle, un groupe-1,4-phénylène, un ou deux atomes d'hydrogène pouvant être substitués par un reste $R^3$, un groupe pyridine-2,5-diyle, un groupe pyridinium-1,4-diyle, un groupe pyridinium-2,5-diyle, l'azote pouvant porter un groupe d'hydrogène, un groupe alkyle de 1 à 20 atomes de carbone ou un groupe phényle substitué ou non substitué, $-CR^4=CR^5-$, $-C\equiv C-$ ;
$D^1$, E, identiques ou différents, représentent A, $B^1$, $C^1$, $-B^1\text{-}A\text{-}C^1-$,

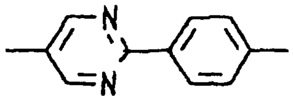 , 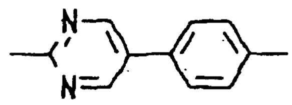 , 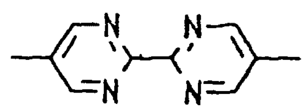 ,

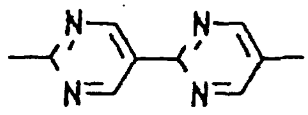 , 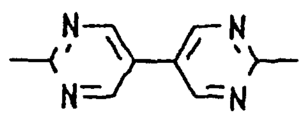 ;

$F^1$, G, identiques ou différents, représentent $D^1$, E, un groupe pyridine-2,5-diyle, un groupe pyridinium-1,4-diyle, un groupe pyridinium-2,5-diyle, l'azote pouvant porter un atome d'hydrogène, un groupe alkyle de 1 à 20 atomes de carbone ou un groupe phényle substitué ou non substitué, un groupe 4-pyridyle ;
$R^1$, $R^2$, $R^3$, $R^4$, $R^5$, identiques ou différents, représentent H, un groupe alkyle non ramifié ou ramifié de 1 à 12 atomes de carbone, un ou plusieurs groupes - $CH_2$- pouvant être substitués par -O-, -CN, -$NR_2^6$ ;
$R^6$ représente H, un groupe alkyle de 1 à 6 atomes de carbone, un groupe phényle ;
a, c, d, e, g identiques ou différents valent 0 ou 1 ;
b, f, identiques ou différents, valent 0, 1 ou 2 ;

à condition que a) la somme a+b+c+d+e+f+g vaille au moins 5 au moins et/ou que b) la somme a+b+c+d+e+f+g vaille au moins 3 et que le groupe $-F^1_a-D^1_b-B^1_c-A_d-C^1_e-E_f-G_g$ contienne au moins 3 groupes pyrimidine-2,5-diyle.

**8.** Composé pyrimidine selon la revendication 7, **caractérisé en ce que** les symboles et indices pour les composés de formule (I) ont les significations suivantes :

A représente un groupe pyrimidine-2,5-diyle, un groupe 1,4-phénylène ;
$B^1$, $C^1$, identiques ou différents, représentent un groupe pyrimidine-2,5-diyle, un groupe 1,4-phénylène, un ou deux atomes d'hydrogène pouvant être substitués par un reste $R^3$, -CH=CH- ;
$D^1$, E, identiques ou différents, représentent A, $B^1$, $C^1$, $-B^1-A-C^1-$,

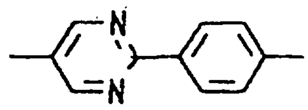 , 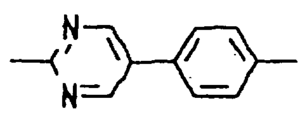 , 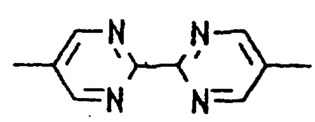 ,

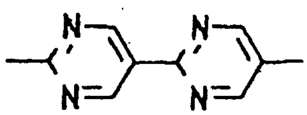 , 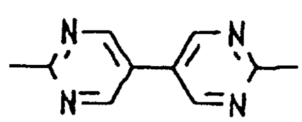 ;

$F^1$, G, identiques ou différents, représentent $D^1$, E ;
$R^1$, $R^2$, $R^3$, identiques ou différents, représentent H, un groupe alkyle non ramifié ou ramifié de 1 à 12 atomes de carbone, un ou plusieurs groupes -$CH_2$- pouvant être substitués par -O-, -CN, -$NR_2^6$ ;
$R^6$ représente H, un groupe alkyle de 1 à 6 atomes de carbone, un groupe phényle ;
a, c, d, e, g, identiques ou différents, valent 0 ou 1 ;
b, f, identiques ou différents, valent 0, 1 ou 2 ;

à condition que a) la somme a+b+c+d+e+f+g vaille de 5 à 16 et/ou que b) la somme a+b+c+d+e+f+g vaille au moins 3 et que le groupe $-F^1_a-D^1_b-B^1_c-A_d-C^1_e-E_f-G_g$ contienne au moins 3 groupes pyrimidine-2,5-diyle.